# EUROPÄISCHE PATENTSCHRIFT

(11) **EP 3 853 863 B1**
(45) Veröffentlichungstag und Bekanntmachung des Hinweises auf die Patenterteilung: **26.02.2025**
(21) Anmeldenummer: 19769128.0
(22) Anmeldetag: 12.09.2019
(51) Int. Cl.: G16H 40/63

(54) **SICHERE STEUERUNG VON DIALYSEGERAETEN**
SECURE CONTROLLING OF DIALYSIS APPARATUSES
COMMANDE SÉCURISÉE D'APPAREILS DE DIALYSE

(30) Priorität: 19.09.2018 DE 102018123011
(43) Veröffentlichungstag der Anmeldung: 28.07.2021
(73) Patentinhaber: Fresenius Medical Care Deutschland GmbH, 61352 Bad Homburg (DE)
(72) Erfinder: MARTERSTOCK, Stefan Konrad, 97337 Dettelbach (DE)
(74) Vertreter: Schwarz, Claudia
(86) Internationale Anmeldenummer: PCT/EP2019/074345
(87) Internationale Veröffentlichungsnummer: WO 2020/058077

(56) Entgegenhaltungen:
- EP-B1- 2 925 381
- DE-A1- 102012 111 523
- US-A1- 2006 143 455

## Beschreibung

Die Erfindung betrifft die sichere Steuerung von medizinischen Geräten, insbesondere Dialysegeräten, von einem entfernten Steuerungsgerät aus.

Dialysegeräte sind Blutbehandlungsgeräte, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin. Verfahren der Hämodialyse, der Hämofiltration und der Hämodiafiltration werden in der Regel mit automatischen Hämodialysegeräten durchgeführt, wie sie beispielsweise von der Anmelderin vertrieben werden. Ein Plasmapheresegerät dient zur Ausführung einer Plasmapherese, einem Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben. Peritonealdialysegeräte dienen zur Durchführung einer Peritonealdialyse, bei der die Bauchhöhle eines Patienten über einen durch die Bauchdecke geführten Katheter mit einer Dialyseflüssigkeit befüllt wird, die ein Konzentrationsgefälle von Blutsubstanzen wie Elektrolyte (beispielsweise Natrium, Kalzium und Magnesium) gegenüber den körpereigenen Flüssigkeiten aufweist. Über das als Membran wirkende Bauchfell (Peritoneum) treten im Körper vorliegende Giftstoffe aus den im Bauchfell verlaufenden Blutgefäßen in die Bauchhöhle über. Nach einigen Stunden wird die sich in der Bauchhöhle des Patienten befindliche, nunmehr mit den aus dem Körper übergetretenen Giftstoffen versetzte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Diese vorstehend genannten und heute auf dem Markt befindlichen Dialysegeräte sind in der Regel autark, d.h. die Programmablauflogik zur Steuerung des Gerätes befindet sich auf dem Gerät selbst. Als Ein- und Ausgabeeinheit ist auf dem Dialysegerät mindestens eine Benutzeroberfläche ausgebildet.

Um die Flexibilität zu erhöhen, ist es wünschenswert, dass die Steuerung der Dialysegeräte auch auf entfernten Steuerungsgeräten erfolgen kann.

Bei medizinischen Geräten sind die Sicherheitsanforderungen jedoch deutlich erhöht im Vergleich zu anderen technischen Geräten. Deshalb muss auch bei einer entfernten Steuerung ein entsprechendes Sicherheitskonzept umgesetzt werden, um einerseits den Missbrauch von zu schützenden Daten (PHI-Daten, protected health data, wie z.B. Patientenbezogenen Daten) und andererseits die unbeabsichtigte und damit den Patienten möglicherweise gefährdende Einflussnahme auf die Steuerung des medizinischen Gerätes (z.B. durch Aufschaltung auf die Steuerleitung) sicher ausschließen zu können.

Systeme zur sicheren Kopplung von Geräten sind bekannt, z.B. aus der US 2006/143455 A1. Diese Schrift offenbart das Senden (304) von einem ersten Gerät (102) sicherer Informationen, wie z. B. eines Bluetooth-Codes, einer PIN oder einer Kombination davon, über eine drahtgebundene Verbindung (260) und das Empfangen (306) von einem zweiten Gerät (104) einer Bestätigung der sicheren Informationen über eine drahtlose Verbindung (105). Weiter umfasst das in dieser Druckschrift beschriebene Verfahren die Kommunikation über eine Verbindung unter Verwendung mindestens eines Teils der sicheren Informationen, um eine sichere Verbindung aufrechtzuerhalten.

Weiter zeigt die DE 10 2012 111 523 A1 ein Dialysebehandlungssystem (1) mit einem Dialysegerät, welches eine Kommunikationsvorrichtung (3) aufweist, welche dazu eingerichtet ist, ein Abfragesignal drahtlos auszusenden und ein Identifikationssignal drahtlos zu empfangen, sowie eine von einem Bediener (100) tragbare Identifikationsvorrichtung (4) zur Identifikation des Bedieners (100), wobei die Identifikationsvorrichtung (4) dazu konfiguriert ist, ein Identifikationssignal drahtlos an das medizinische Gerät (2) zu senden.

Aufgabe der vorliegenden Erfindung ist es deshalb, ein verbessertes Sicherheitskonzept für ein medizinisches Gerät oder eine Gruppe dieser Geräte bereitzustellen, das einerseits eine erhöhte Flexibilität der Bedienung und Wartung ermöglicht und dabei andererseits auch den erhöhten Sicherheitsanforderungen durch strukturelle bauliche Maßnahmen entsprechen kann.

Diese Aufgabe wird erfindungsgemäß durch Geräte, Systeme und Verfahren gemäß den beiliegenden, einander nebengeordneten Patentansprüchen gelöst.

Im Folgenden wird die Erfindung anhand der vorrichtungsgemäßen Aufgabenlösung, und somit anhand des Steuerungsgerätes beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die verfahrensbasierten Ansprüche mit den Merkmalen weitergebildet sein, die in Zusammenhang mit dem Steuerungsgerät beschrieben und/oder beansprucht sind und umgekehrt. Die entsprechenden funktionalen Merkmale des Verfahrens werden dabei durch entsprechende gegenständliche Module, insbesondere durch elektronische Hardware-Module oder Mikroprozessor-Module, des Systems bzw. des Produktes ausgebildet und umgekehrt. So kann z.B. das Erfassen eines Initialisierungssignals durch einen entsprechenden Signalgeber ausgeführt werden, der z.B. eine Schaltfläche auf einer Oberfläche ausgebildet sein kann.

Gemäß einem ersten Aspekt betrifft die Erfindung ein Steuerungssystem nach Anspruch 1.

Das Steuerungssystem kann ein Steuerungsgerät umfassen zum Aufbau einer gesicherten Kommunikationsverbindung zum Steuern von zumindest einem entfernten medizinischen Gerät, insbesondere einem Dialysegerät. Das Steuerungsgerät ist somit getrennt von oder außerhalb von dem medizinischen Gerät angeordnet und umfasst zumindest eine Steuerungsinstanz und in der Regel mehrere Steuerungsinstanzen, die jeweils zur Steuerung zumindest eines medizinischen Gerätes beauftragt sind. Dazu ist das Steuerungsgerät weiter ausgebildet mit:
- einer (steuerungsgeräteseitigen bzw. serverseitigen) Kommunikationseinrichtung zur Kommunikation mit dem medizinischen Gerät;
- einem integrierten Schaltkreisbaustein, der ausgebildet ist, ein Sicherungsprotokoll zum Herstellen einer gesicherten Kommunikationsverbindung zur Steuerung des medizinischen Gerätes zu implementieren, und wobei das Sicherungsprotokoll Identifikationsnachrichten zur eineindeutigen Identifikation des medizinischen Gerätes und zur eineindeutigen Identifikation der jeweiligen Steuerungsinstanz auf dem Steuerungsgerät austauscht.

Gemäß einer bevorzugten Ausführungsform der Erfindung wird das Ausführen des Sicherungsprotokolls auf dem Steuerungsgerät durch Erfassen einer Initialisierungsnachricht auf dem Steuerungsgerät initialisiert. Die Initialisierungsnachricht wird in der Regel von dem medizinischen Gerät an das Steuerungsgerät gesendet. Damit geht die Initiierung des Verfahrens zum Aufbau einer gesicherten Kommunikationsverbindung vom medizinischen Gerät aus. Dies hat den technischen Hintergrund, dass sozusagen die Hoheit über die Steuerungssituation auf dem zu steuernden medizinischen Gerät liegt. Damit kann die Sicherheit erhöht werden, indem entfernt ausgelöste Kompromittierungsversuche unterbunden werden können.

Gemäß einer weiteren, bevorzugten Ausführungsform der Erfindung umfasst das Steuerungsgerät eine Benutzerschnittstelle. Die Benutzerschnittstelle dient zum Erfassen von Steuersignalen zur Steuerung des medizinischen Gerätes. Kumulativ oder alternativ kann die Benutzerschnittstelle nicht nur zur Dateneingabe (von Steuerbefehlen) dienen, sondern auch zur Datenausgabe von Zustandsdaten des medizinischen Gerätes. So kann insbesondere ein Geräte- und/oder Behandlungszustand ausgegeben werden. Es kann sich z.B. um eine direkt auf dem Steuerungsgerät ausgebildete Oberfläche, insbesondere eine grafische Oberfläche handeln. Alternativ ist es auch möglich, die Benutzerschnittstelle in einem separaten Gerät und nicht auf dem Steuerungsgerät auszubilden. Damit kann die Flexibilität zur Gerätekopplung zum Aufbau eines gesicherten Kommunikationskanals gesteigert werden. In diesem Fall steht die separate Benutzeroberfläche bzw. Benutzerschnittstelle in Datenaustausch mit dem Steuerungsgerät.

In einem weiteren Aspekt wird die Aufgabe gelöst durch ein Sicherungsverfahren nach Anspruch 3.

Es kann vorgesehen sein, dass das medizinisches Gerät über ein entferntes Steuerungsgerät gesteuert wird. Das medizinische Gerät umfasst eine clientseitige Kommunikationsreinrichtung, die zum Datenaustausch mit einer serverseitigen Kommunikationseinrichtung des Steuerungsgerätes ausgebildet ist. Weiterhin umfasst es einen integrierten Schaltkreisbaustein, der ausgebildet ist, ein Sicherungsprotokoll zum Herstellen einer gesicherten Kommunikationsverbindung zu dem Steuerungsgerät zu implementieren, und wobei das Sicherungsprotokoll Identifikationsnachrichten zur eineindeutigen Identifikation des medizinischen Gerätes und zur eineindeutigen Identifikation einer jeweiligen Steuerungsinstanz auf dem Steuerungsgerät austauscht, wobei jeweils eine Steuerungsinstanz zur dedizierten Steuerung eines bestimmten medizinischen Gerätes bestimmt ist.

Das Steuerungssystem zum Aufbau einer gesicherten Kommunikationsverbindung zum Steuern zumindest eines medizinischen Gerätes, insbesondere eines Dialysegerätes, mit einem entfernten Steuerungsgerät, umfasst:
- Das zu steuernde medizinische Gerät;
- Ein Steuerungsgerät, wie vorstehend beschrieben, wobei die beteiligten Geräte über zumindest ein Netzwerk (drahtlos und/oder drahtgebunden) in Datenaustausch stehen.

In einer vorteilhaften Ausführungsform der Erfindung kann das Steuerungssystem zusätzlich optional eine Benutzerschnittstelle umfassen, die entweder auf dem Steuerungsgerät oder extern und separat bzw. getrennt davon ausgebildet sein kann, z.B. auf einer mobilen Kommunikationseinrichtung, wie einem Mobilfunkgerät oder einem anderen elektronischen Gerät oder Endgerät. Dabei steht das separate Gerät mit der Benutzerschnittstelle in bidirektionalem Datenaustausch mit dem Steuerungsgerät und/oder mit dem Steuerungssystem. Damit kann die Flexibilität erhöht werden. In einer vorteilhaften Ausgestaltung ist es sichergestellt, dass die externe Benutzerschnittstelle nur dann über das medizinische Gerät mit dem Steuerungsgerät kommunizieren kann, wenn sich die externe Benutzerschnittstelle im räumlichen Umfeld bzw. in der Nähe zum medizinischen Gerät befindet. Dies kann z.B. über eine Positionsermittlung ausgeführt werden, so wie in der Patentschrift EP2925381B1 näher beschrieben. Damit wird der nicht-autorisierte Zugang über Funknetzwerke weiter erschwert.

Wie oben bereits erwähnt, wird die Aufgabe gelöst durch ein Sicherungsverfahren für zumindest ein medizinisches Gerät, insbesondere für ein Dialysegerät gemäß Anspruch 3 und insbesondere durch ein Sicherungsverfahren, das in einem Steuerungssystem verwendet wird. Das Sicherungsverfahren dient zum Aufbau einer gesicherten Kommunikationsverbindung zwischen dem medizinischen Gerät und einem entfernt angeordneten Steuerungsgerät, wobei das Steuerungsgerät zumindest eine Steuerungsinstanz umfasst, die jeweils zur Steuerung zumindest eines medizinischen Gerätes beauftragt ist. Das Sicherungsverfahren kann in einem Steuersystem verwendet werden und umfasst erste Abschnitte, die auf dem medizinischen Gerät und zweite Abschnitte, die auf dem Steuerungsgerät ausgeführt werden. Beide Abschnitte zusammen bilden ein verteiltes Verfahren zum Ausführen eines Sicherungsprotokolls auf dem Steuerungsgerät und dem medizinischen Gerät zum Herstellen einer sicheren Kommunikationsverbindung zur (späteren) Steuerung des medizinischen Gerätes. Das Sicherungsprotokoll tauscht Identifikationsnachrichten zur eineindeutigen Identifikation des medizinischen Gerätes und zur eineindeutigen Identifikation der jeweiligen Steuerungsinstanz auf dem Steuerungsgerät mit dem medizinischen Gerät aus.

In einer vorteilhaften Ausführungsform der Erfindung kann das Sicherungsverfahren vor der Ausführung des Sicherungsprotokolls zusätzlich den Verfahrensschritt umfassen:
- Erfassen eines Triggersignals auf dem medizinischen Gerät, um die Ausführung des Sicherungsprotokolls auszulösen. Damit wird sichergestellt, dass die Initiierung des Kopplungsprozesses von dem medizinischen Gerät ausgeht. Dies kann die Sicherheit verbessern.

In einer vorteilhaften Ausführungsform der Erfindung umfasst das Ausführen des Sicherungsprotokolls folgende Schritte:
- Senden einer Initialisierungsnachricht mit einer eindeutigen Geräteidentifikation für das medizinische Gerät von dem medizinischen Gerät an das Steuerungsgerät und insbesondere an alle Steuerungsinstanzen auf dem Steuerungsgerät; sollten mehrere Steuerungsgeräte in dem System zum Einsatz kommen, kann die Initialisierungsnachricht auch an alle Steuerungsgeräte gesendet werden. Dies kann vorzugsweise in Form einer Broadcast-Nachricht ausgeführt werden.
- Empfangen der Initialisierungsnachricht auf dem Steuerungsgerät;
- Seitens des Steuerungsgerätes: Senden einer ersten Bestätigungsnachricht an das medizinische Gerät mit einer Identifikation der zugeordneten Steuerungsinstanz in Antwort auf die empfangene Initialisierungsnachricht;
- Empfangen der ersten Bestätigungsnachricht auf dem medizinischen Gerät und
- Seitens des medizinischen Gerätes: Senden einer zweiten Bestätigungsnachricht in Antwort auf die empfangene erste Bestätigungsnachricht an das Steuerungsgerät.

In einer vorteilhaften Ausführungsform der Erfindung wird das Steuerungsgerät nach dem Empfangen der Initialisierungsnachricht in Antwort auf die empfangene Initialisierungsnachricht konfiguriert. Das Konfigurieren kann beispielsweise ein Bestimmen einer spezifischen Steuerungsinstanz für das jeweilige medizinische Gerät und damit dessen Zuweisung (allocation process) zum Gerät umfassen. Das Konfigurieren kann ein Bestimmen von Parametern (Parametrisierung für das spezifische medizinische Gerät) umfassen. Dies ist insbesondere dann vorteilhaft, wenn das Steuerungsgerät mehrere medizinische Geräte parallel steuern soll bzw. mehrere sichere Kommunikationsverbindungen zwischen dem Steuerungsgerät und unterschiedlichen Medizingeräte aufzubauen sind. Damit kann der technische Vorteil erreicht werden, dass mehrere medizinische Geräte über eine entsprechende Netzwerkverbindung parallel oder gleichzeitig bedient und gesteuert werden können. Folglich wird die zentrale und einheitliche Steuerung einer Gruppe von Geräten (z.B. unter Verwendung eines in einer Datenbank hinterlegten vorkonfigurierbaren Steuerschema) vereinfacht.

Mit anderen Worten können mehrere Steuerungsinstanzen (für mehrere medizinische Geräte) auf dem Steuerungsgerät ausgebildet sein. Vorzugsweise wird dann die Initialisierungsnachricht eine Broadcastnachricht an alle Steuerungsinstanzen des Steuerungsgerätes sein. Alternativ oder kumulativ können auch mehrere Steuerungsgeräte in dem gleichen Netzwerk eingebunden sein.

In einer anderen, vorteilhaften Ausführungsform der Erfindung umfasst die Initialisierungsnachricht einen Ausstattungscode, der die technische Ausstattung des medizinischen Gerätes repräsentiert. Der Begriff Ausstattung bezieht sich auf die technische Ausrüstung des Gerätes und kann folgende Parameter umfassen: eine Version des Gerätes, implementierte Hardware und/oder Software, Module des Gerätes und/oder Einsatzzweck und/oder Leistungsdaten des Geräts und oder weitere technische Parameter. Alternativ oder kumulativ kann die Initialisierungsnachricht auch Kalibrierdaten umfassen.

In einer anderen, vorteilhaften Ausführungsform der Erfindung wird nach Ausführung des Sicherungsprotokolls ein Kopplungsergebnis auf einer Benutzerschnittstelle des Steuerungsgerätes und/oder des medizinischen Gerätes angezeigt. Das Kopplungsergebnis kann eine Angabe zum Erfolg des Sicherungsverfahrens bzw. des Kopplungsprozesses kennzeichnen. Das Kopplungsergebnis kann in Form einer Nachricht ausgebildet sein, um z.B. zu signalisieren, dass das gesicherte Pairing erfolgreich war oder fehlgeschlagen ist.

In einer anderen, vorteilhaften Ausführungsform der Erfindung wird nach Ausführung des Sicherungsprotokolls ein Kopplungsergebnis mittels einer Aktvierung eines Hardwarebausteins signalisiert. Der Hardwarebaustein kann ausgebildet sein zumindest zwei Zustände parallel darzustellen: einen ersten Zustand einer erfolgreichen Kopplung und einen zweiten Zustand einer fehlerhaften oder nicht erfolgreichen Kopplung. Dies kann insbesondere in Form einer Ampelsignalgebung erfolgen (z.B. in Form einer elektronischen Ampel: Grün für erfolgreiche Kopplung und Aufbau eines gesicherten Kommunikationskanals und rot für fehlgeschlagene Kopplung).

In einer anderen, vorteilhaften Ausführungsform der Erfindung umfasst die Ausführung des Sicherungsprotokolls:
- Seitens des Steuerungsgerätes: Auswerten, ob eine zweite Bestätigungsnachricht in Antwort auf eine empfangene erste Bestätigungsnachricht auf dem Steuerungsgerät innerhalb einer vordefinierten Timeout-Zeitspanne empfangen wurde, und verneinendenfalls: Ausgeben einer Fehlkopplungsnachricht zumindest auf dem Steuerungsgerät (und/oder auf weiteren elektronischen Instanzen, insbesondere auf dem medizinischen Geräte) und/oder bejahendenfalls: Ausgaben einer Kopplungsnachricht (= die ein erfolgreiches Ergebnis signalisiert) auf dem Steuerungsgerät. Im Anschluss kann der Aufbau der gesicherten Kommunikationsverbindung erfolgen. Sobald diese aufgebaut ist, kann das medizinische Geräte vom Steuerungsgerät aus auf gesicherte Weise ferngesteuert werden.

Beschrieben wird weiter ein Server-Schutzsystem für ein Steuerungsgerät, das ein entferntes medizinisches Gerät, insbesondere ein Dialysegerät, über eine gesicherte Kommunikationsverbindung steuert. Das Server-Schutzsystem umfasst dazu:
- Eine Prüfeinheit, die ausgebildet ist, eine zwei Aspekte umfassende Prüfung auszuführen, nämlich, kontinuierlich zu überprüfen, ob die gesicherte Kommunikationsverbindung fortbesteht und der Behandlungsablauf korrekt, so dass kein Patientengefährdungszustand besteht und falls ja (also falls die gesicherte Kommunikationsverbindung fortbesteht und falls der Behandlungsablauf am Dialysegerät korrekt ist), eine Freigabeeinheit mit zumindest einem im Folgenden genannten Befehl instruiert. Das Prüfen, ob der Behandlungsablauf korrekt ist, wird vorzugsweise mittels eines Prüfalgorithmus' auf Basis der über die gesicherte Kommunikationsverbindung ausgetauschten Daten ausgeführt. Das Prüfen kontrolliert den Verfahrensablauf am Dialysegerät auf Korrektheit. Ein erster Aspekt des Prüfens bezieht sich somit auf die Korrektheit der Kommunikation (Datenintegrität) und ein zweiter Aspekt bezieht sich auf die Prüfung der Dateninhalte selbst (z.B. Temperatur unter Grenzwert ...). In beiden Fällen würde ein Freigabesignal unterbleiben, falls eine der beiden Prüfungen nicht bestanden wird. In einer vorteilhaften Ausführungsform der Erfindung kann der Prüfalgorithmus' ausschließlich auf dem medizinischen Gerät ausgeführt werden. Damit kann die Sicherheit erhöht werden.
- Der Freigabeeinheit, die bei Instruktion durch die Prüfeinheit ein Freigabesignal an das medizinische Gerät sendet oder die Versendung des Freigabesignals durch eine andere elektronische Einheit auslöst.

Beschrieben wird weiterhin ein Client-Schutzsystem für ein medizinisches Gerät, insbesondere ein Dialysegerät, das von einem Steuerungsgerät über eine gesicherte Kommunikationsverbindung gesteuert wird. Das Client-Schutzsystem umfasst dazu:
- Eine Überwachungseinheit, die ausgebildet ist, kontinuierlich und automatisch zu überwachen, ob das zumindest eine Freigabesignal innerhalb einer vordefinierten Fehlertoleranzzeit empfangen wurde. Optional kann zusätzlich ein zweiter Aspekt überwacht werden, nämlich, ob der Behandlungsablauf korrekt ist, und falls die zwei Aspekte umfassende Überwachung in zumindest einem Aspekt nicht erfolgreich war, eine Transferlogik mit einem entsprechenden Befehl (wie folgt beschrieben) instruiert;
- Die Transferlogik, die dazu ausgebildet ist, das medizinische Gerät automatisch in einen sicheren Gerätezustand zu überführen, wenn sie von der Überwachungseinheit entsprechend dem Ergebnis der Überwachung instruiert worden ist, z.B. über ein unterbliebenes weiteres Freigabesignal der Überwachungseinheit im Gutfall oder über ein Sperrsignal im Schlechtfall der Überwachung (also falls zumindest einer der Aspekte nicht auf einen erfolgreichen Zustand hinweist).

Die Transferlogik ist ein elektronisches Modul (Hardware und/oder Software), das auf dem medizinischen Gerät implementiert ist und das Gerät von einem Betriebszustand in einen sicheren Gerätezustand überführt und umgekehrt. Im übertragenen Sinn fungiert die Transferlogik somit als Schalter. Die Transferlogik wird durch entsprechende Eingangssignale, insbesondere ein Sperrsignal oder ein weiteres Freigabesignal, gesteuert. Der sichere Gerätezustand kennzeichnet sich dadurch, dass keine die Patientensicherheit gefährdenden Maßnahmen ausgelöst werden können. In einem Spezialfall kann das medizinische Gerät auch teilweise oder vollständig deaktiviert werden, wenn es in den sicheren Gerätezustand transferiert werden soll. In einer bevorzugten Ausführungsform der Erfindung ist es im Vorfeld konfigurierbar, welche Funktionalitäten in dem sicheren Gerätezustand betrieben bzw. ausgeführt werden können sollen (z.B. Blutfluss zum Patienten stoppen). So werden gewöhnlich die arterielle und venöse Klemme zum Patienten geschlossen, so dass sichergestellt ist, dass keine Energie und/oder kein Fluidtransport zwischen dem Dialysegerät und dem Patienten mehr möglich ist. In anderen Fällen kann es auch konfiguriert sein, dass die Blutpumpe gestoppt bzw. das Dialysegerät auch abgeschaltet wird (z.B. bei zu hohen internen, elektrischen Spannungen). Es gibt noch weitere Fälle, bei denen bspw. eine Blutrückgabe erlaubt ist.

In einer bevorzugten Ausführungsform der Erfindung ist es bei dem Client-Schutzsystem vorgesehen, dass die Überwachungseinheit das medizinische Gerät oder eine andere elektronische Instanz auf dem medizinischen Gerät (z.B. eine Sendeeinheit und/oder die client-seitige Kommunikationseinrichtung) instruiert, Status-Datenpakete über die gesicherte Kommunikationsverbindung an das Server-Schutzsystem zu senden, falls zumindest ein Freigabesignal innerhalb einer vordefinierten Fehlertoleranzzeit empfangen wurde.

Beschrieben wird weiterhin ein Betriebsverfahren für ein verteiltes Schutzsystem, umfassend ein Client-Schutzsystem, das auf einem medizinischen Gerät, insbesondere einem Dialysegerät, implementiert ist und weiter umfassend ein Server-Schutzsystem, das auf einem entfernt vom medizinischen Gerät angeordneten Steuerungsgerät implementiert sein kann, und bei dem kontinuierlich überprüft wird, ob eine gesicherte Kommunikationsverbindung zwischen dem medizinischen Gerät und dem Steuerungsgerät fortbesteht, umfassend folgende, automatisch ausgeführte Schritte:
- Auf dem Server-Schutzsystem: Signalaustausch-basiertes Prüfen, ob eine gesicherte Kommunikationsverbindung zwischen dem medizinischen Gerät und dem Steuerungsgerät fortbesteht und der Behandlungsablauf korrekt bzw. ob kein Patientengefährdungszustand besteht und falls das Prüfen erfolgreich war (gesicherte Kommunikationsverbindung besteht fort und Behandlungsablauf ist korrekt bzw. es liegt kein Patientengefährdungszustand vor): Zyklisches Senden zumindest eines Freigabesignals an das medizinische Gerät;
- Seitens des Client-Schutzsystem: Vollautomatisches, computer-implementiertes Überwachen, ob das zumindest eine Freigabesignal innerhalb einer vordefinierten Fehlertoleranzzeit empfangen wurde und verneinendenfalls: Automatisches Überführen des medizinischen Gerätes in einen sicheren Gerätezustand.

In einer bevorzugten Ausführungsform der Erfindung kann das signalaustausch-basierte Prüfen ausgeführt werden, indem das medizinische Gerät zyklisch Status-Datenpakete an das Schutzsystem sendet und das Schutzsystem den konsekutiv korrekten Empfang der Status-Datenpakete analysiert. Dies hat den technischen Vorteil, dass die ohnehin zu übertragenden Datenpakete, wie z.B. Sensordaten oder Statusdaten verwendet werden können, um die Grundlage einer serverseitigen Prüfung zu sein.

In einer weiteren vorteilhaften Ausführungsform des erfindungsgemäßen Betriebsverfahrens können die Status-Datenpakte einen Zeitstempel oder eine Sequenznummer (als fortlaufenden Nummer) umfassen. Dies ist insbesondere dann hilfreich, wenn die Datenpakete ansonsten nicht unterscheidbar hinsichtlich ihres Zeitsignals wären.

In einer weiteren vorteilhaften Ausführungsform des Betriebsverfahren kann das Server-Schutzsystem auf dem Steuerungsgerät, vorzugsweise in einem geschützten Speicherbereich des Steuerungsgerätes, implementiert sein. Alternativ kann das Server-Schutzsystem auch auf einem hardwaretechnisch getrennt realisierten zweiten Steuerungsgerät implementiert sein. Damit kann die Sicherheit verbessert werden.

In einer weiteren vorteilhaften Ausführungsform des Betriebsverfahren wird selbiges vollständig automatisch und computer-implementiert und insbesondere ohne Benutzerinteraktion ausgeführt. Damit kann das Betreiben des Schutzsystems sicherer gemacht werden, indem Fehleingaben eines Anwenders ausgeschlossen sind.

Beschrieben wird weiterhin ein verteilt realisiertes Schutzsystem für ein medizinisches Gerät, insbesondere ein Dialysegerät, das von einem entfernten Steuerungsgerät gesteuert wird, und wobei das Schutzsystem umfasst:
- Ein Server-Schutzsystem, wie vorstehend beschrieben und
- Ein Client-Schutzsystem, wie ebenfalls vorsehend beschrieben.

Ein erster Abschnitt des Betriebsverfahrens kann auf dem Steuerungsgerät und ein zweiter Abschnitt kann auf dem medizinischen Gerät ausgeführt werden. Der zweite Abschnitt kann auch auf einer separaten Instanz, insbesondere einem Schutzsystem implementiert sein. Es ist auch möglich, dass das Schutzsystem zumindest teilweise auf dem Steuerungsgerät implementiert ist. Um die Sicherheit zu gewährleisten, muss sichergestellt sein, dass im Dialysegerät immer ein Teil des Schutzsystems realisiert sein muss, zumindest ein Bauteil, das nach der Art eines "Watchdog" betrieben wird oder eine Überwachungseinheit.

In einer bevorzugten Ausführungsform der Erfindung kann bei dem Betriebsverfahren das Prüfen (ob das zumindest eine Freigabesignal gültig empfangen wurde) ausgeführt werden, indem das medizinische Gerät zyklisch und/oder ereignisbasiert (also nach vordefinierbaren Ereignissen) Status-Datenpakete an das Schutzsystem sendet und das Schutzsystem den konsekutiv korrekten Empfang der Status-Datenpakete überwacht. "Konsekutiv korrekt" meint in diesem Zusammenhang, dass eine Abfolge von elektronischen Nachrichten, nämlich den Status-Datenpaketen, überwacht wird. Die gesendete Abfolge muss der empfangenen Abfolge entsprechen. Sobald ein Unterschied festgestellt wird (z.B. wenn die gesendete Abfolge 1- 3 - 5- 7 - ..ist, und die empfangene Abfolge 1 - 5 - 3 - 7 - ist), dann liefert der Prüfvorgang bzw. das Prüfen einen Fehler, obwohl im Endergebnis alle Status-Datenpakete empfangen werden konnten. Dies lässt z.B. vermuten, dass die Netzwerkverbindung kurzzeitig und insbesondere während des Übertragungsprozesses von Paket 5 unterbrochen war und das Versenden des Datenpaketes 5 zu einem späteren Zeitpunkt nachgeholt worden ist.

In einer bevorzugten Ausführungsform des Betriebsverfahrens können die Status-Datenpakte einen Zeitstempel umfassen. Damit kann die Qualität der Prüfung, ob ein Freigabesignal vorgelegen hat und insbesondere, ob das Freigabesignal gültig war, verbessert werden, indem nicht nur die Abfolge (Sequenz der Signale), sondern auch deren Zeitstempel überprüft werden. Die Zeitstempel müssen eine kontinuierliche Abfolge darstellen, z.B. bei einem 1-Minuten- Raster muss die Sequenz sein: Minute 1, Minute 2, Minute 3, Minute 4 etc. Wenn z.B. erfasst wird, dass der Zeitstempel 1 auf 3 erfasst wurde, ist dies ein Indiz, dass die Abfolge unterbrochen war und deutet auf eine (möglicherweise) vorübergehende Unterbrechung des gesicherten Kommunikationskanals hin. Dieses Zwischenergebnis kann auf der Benutzerschnittstelle ausgegeben werden.

In einer weiteren bevorzugten Ausführungsform des Betriebsverfahrens kann das Schutzsystem auf dem Steuerungsgerät, vorzugsweise in einem geschützten Speicherbereich, implementiert sein. Alternativ kann das Schutzsystem als separates elektronisches Modul (Hardware und/oder Software-Modul) über eine entsprechende Datenverbindung mit dem Steuerungsgerät verbunden und zusätzlich zum selbigen in das System eingebunden sein.

Beschrieben wird weiterhin ein verteilt realisiertes Schutzsystem für ein medizinisches Gerät, insbesondere für ein Dialysegerät, das von einem entfernten Steuerungsgerät gesteuert wird, und wobei das Schutzsystem nach einem vorstehend beschriebenen Betriebsverfahren betrieben wird. Dabei kann die Benutzerschnittstelle des Steuerungsgerätes als externe (z.B. mobile) Instanz zugeschaltet sein.

Grundsätzlich und um Sicherheitsvorgaben einzuhalten, akzeptiert ein Dialysegerät nur jeweils eine Steuerungsinstanz und eine Schutzsysteminstanz. Durch das Verwenden des Triggersignals ist sichergestellt, dass die Kopplung zwischen Dialysegerät und Steuerungsgerät also der Aufbau der gesicherten Kommunikationsverbindung nur außerhalb einer medizinischen Behandlung (Dialysebehandlung) möglich ist. Dazu kann auch die Überwachungseinheit verwendet werden, um zu blockieren, dass nicht während einer Behandlung in den Pairing-Modus gewechselt werden kann.

Die erste Bestätigungsnachricht, die die Steuerungsinstanz an das medizinische Gerät sendet ist vorzugsweise verschlüsselt. Dies kann unter Zuhilfenahme eines "Secrets" erfolgen, das werkseitig vom Hersteller des Gerätes bereitgestellt wird. Beispielsweise erwartet das Dialysegerät in der ersten Bestätigungsnachricht eine Prüfzahl, die von der Steuerungsinstanz auf Basis des Secrets und der zuzuordnenden Identifikationsnummer des medizinischen Gerätes ermittelt wird. Somit kann auch sichergestellt werden, dass das medizinische Gerät nur die für sich bestimmten Bestätigungsnachrichten akzeptiert und nicht die des Nachbargeräts. Die Verarbeitung von Bestätigungsnachrichten von unautorisierten Instanzen/Hackern wird somit verhindert.

Das Secret muss jedoch nicht zwingend werksseitig implementiert werden, sondern kann bspw. auch durch einen Servicetechniker via Interaktion d.h. via Software-Download oder steckbarer Hardware auf dem medizinischen Gerät eingespielt werden und ebenfalls durch den Servicetechniker in das Steuerungsgerät eingespielt oder manuell eingegeben werden (Tastatur, Voice). Hierdurch ist eine größere Vielfalt bei den Secrets möglich. Ebenfalls ist es möglich, dass in dem medizinischen Gerät eines von mehreren vordefinierten Secrets werksseitig hinterlegt ist, aber der Servicetechniker dieses am Steuerungsgerät einstellen, konfigurieren oder komplett bzw. teilweise eingeben muss.

Es kann vorkonfiguriert sein, dass ein Triggersignal nur durch eine Bedienung eines autorisierten Personals ausgelöst werden kann, um die Sicherheit weiter zu erhöhen.

Während des Aufbaus der gesicherten Kommunikationsverbindung (Pairing-Vorgang) werden vorzugsweise Broadcast-Messages verwendet. Die Broadcast-Nachrichten enthalten die jeweiligen Kopplungspartner, sodass alle Netzwerkteilnehmer im Bilde sind, ob sie zum Aufbau der gesicherten Kommunikationsverbindung akzeptiert wurden oder nicht und welches Gerät der Pairings-Partner ist. Durch Anzeigen des Kopplungsergebnisses unter Anzeige der zugeordneten Geräte-Identifikationsnummer kann z.B. autorisiertes Personal (Servicetechniker) die Korrektheit des Vorgangs am Ende noch einmal überprüfen. Dies erhöht die Sicherheit und die Transparenz. Sobald die Verschlüsselung aktiv ist (ab erster Bestätigungsnachricht stellen die anderen Teilnehmer dies fest und können eine entsprechenden Meldung ausgeben) werden von da an Unicast Nachrichten verwendet.

Die Geräte-Identifikationsnummer aus der Initialisierungsnachricht kann bereits zu Beginn auf der Benutzerschnittstelle des Steuerungsgerätes dem Servicetechniker angezeigt werden, um einen falschen Pairing-Vorgang frühzeitig abbrechen zu können.

Vorzugsweise ist das Netzwerk ein privates d.h. physikalisch eigenständiges Kliniknetzwerk. Insbesondere kann es ein kabelgebundenes Netzwerk oder ein Funknetzwerk mit begrenzter Funkreichweite sein.

In einer Ausführungsform der Erfindung ist es vorgesehen, dass Kalibrierdaten im Gerät bspw. ebenfalls am Ende des Kopplungsvorgangs oder während der Instanziierung der Steuerungsinstanz vom medizinischen Gerät an das Steuerungsgerät übertragen werden können.

Im Folgenden werden die Begrifflichkeiten der Anmeldung näher definiert.

Das Steuerungsgerät ist ein elektronisches Endgerät mit einem Prozessor oder Microprozessor, der eine Applikation zur Steuerung des medizinischen Gerätes ausführt. Das Steuerungsgerät kann direkt oder indirekt mit einer Benutzerschnittstelle ausgebildet sein. "Indirekt" meint in diesem Zusammenhang, dass die Benutzerschnittstelle auch auf einem externen Gerät ausgebildet sein kann (z.B. Mobilfunkgerät), das über eine entsprechende Netzwerkverbindung (z.B. WLAN) oder Schnittstelle mit dem Steuerungsgerät in Datenaustausch steht. Das Steuerungsgerät kann als Server ausgebildet sein. Das Steuerungsgerät kann insbesondere als physikalischer und/oder virtueller Server ausgebildet sein und von einem Betriebssystem (operating system) betrieben werden. Das Steuerungsgerät kann zur (zentralen) Steuerung einer Mehrzahl von medizinischen Geräten ausgebildet sein. Das Steuerungsgerät kann dazu mit zumindest einer Steuerungsinstanz ausgebildet sein. Die Steuerungsinstanz kann z.B. eine Applikation sein, die zur Steuerung eines spezifischen medizinischen Gerätes bestimmt ist. Die Zuordnung zwischen Steuerungsinstanz und zu steuerndem medizinischen Gerät ist eindeutig. Mit anderen Worten wird ein medizinisches Gerät von genau einer Steuerungsinstanz gesteuert. Dies dient dazu, die erhöhten Sicherungsanforderungen zu erfüllen.

Es soll eine gesicherte Kommunikationsverbindung zwischen Steuerungsgerät und dem zu steuernden medizinischen Gerät aufgebaut werden. Dies bedeutet, dass der Kommunikationskanal zusätzlich abgesichert ist. Dies wird erreicht, indem ein vordefiniertes Datenaustauschprotokoll mit auszutauschenden Nachrichten hinterlegt ist, und überprüft wird, ob das Datenaustauschprotokoll mit der Abfolge von vordefinierten Nachrichten (gemäß Protokoll) eingehalten worden ist. Über die gesicherte Kommunikationsverbindung zwischen Steuerungsgerät und dem zu steuernden medizinischen Gerät können Steuerbefehle an das medizinische Gerät und/oder Zustandsdaten an das Steuergerät übertragen werden. Die gesicherte Kommunikationsverbindung kann somit vorzugsweise zum bidirektionalen Datenaustausch ausgebildet sein.

Das medizinische Gerät kann ein elektronisches Gerät sein, das medizinische Funktionen bereitstellt. Es kann sich insbesondere um ein Dialysegerät oder um ein anderes Blutbehandlungsgerät handeln.

Um überhaupt einen Datenaustausch zwischen dem Steuerungsgerät und dem medizinischen Gerät bereitzustellen, ist das Steuerungsgerät mit einer Kommunikationseinrichtung zur vorzugsweise drahtgebundenen Kommunikation mit dem medizinischen Gerät ausgebildet. Die auf dem Steuerungsgerät befindliche Kommunikationseinrichtung wird auch als serverseitige Kommunikationseinrichtung bezeichnet. In Entsprechung dazu weist jedes medizinische Gerät ebenfalls eine Kommunikationseinrichtung auf, die als clientseitige Kommunikationseinrichtung bezeichnet werden kann.

In einer weiteren bevorzugten Ausführungsform der Erfindung umfasst das Steuerungssystem nicht nur das medizinische Gerät und das Steuerungsgerät, sondern zusätzlich noch ein drittes elektronisches Gerät, das quasi als mobile Benutzerschnittstelle eines Endgerätes für das Steuerungsgerät agiert. Damit kann die Flexibilität der Bedienung noch gesteigert werden, Um jedoch auch die Sicherheit gewährleisten zu können, soll auf dem Steuerungsgerät automatisch erfasst werden, wenn sich das dritte mobile Benutzerschnittstellengerät nähert. Deshalb ist es in einer bevorzugten Ausführungsform der Erfindung vorgesehen, dass das medizinische Gerät eine räumliche Näherung des mobilen Endgeräts (Benutzerschnittstelle), einer Kommunikationseinrichtung oder eines anderen Bauteils des Steuerungsgerätes detektiert (und zusätzlich meldet, um welches Gerät es sich handelt, mit Angabe einer eindeutigen Adresse). Dazu können Methoden angewendet werden, wie sie in dem EP Patent Nr. 2925381 beschrieben sind, dessen Offenbarungsgehalt hiermit referenziert wird. Damit wird es möglich, z.B. Tablets als mobile Bedienerschnittstelle zusätzlich durch eine örtliche Ortung abzusichern. Eine Kopplung bzw. ein Pairing wird z.B. nur möglich, wenn sich die mobile Bedienerschnittstelle (Handy/ Tablet) im Umkreis von einer vorkonfigurierbaren Anzahl von Metern vom Dialysegerät befindet. Diese zusätzliche Absicherung über eine Positionserfassung kann auch in anderen Ausführungsform der Erfindung angewendet werden, z.B. auch als Zusatzfunktion zum Pairing mit einer Dialysemaschine, ohne die Verwendung eines Steuerungsgerätes.

Die serverseitige und die clientseitige Kommunikationseinrichtung dienen zum Datenausaustausch über zumindest ein Netzwerk (z.B. LAN, WLAN, drahtlose Kommunikationsmittel im Nahfeldbereich, wie Bluetooth, NFC - nearfield communication, DSRC dedicated short range communication, oder auch drahtgebunden Kommunikationsmittel) und insbesondere zur Ausführung des Sicherungsprotokolls.

Der integrierte Schaltkreisbaustein ist ausgebildet, ein Sicherungsprotokoll zum Herstellen der gesicherten Kommunikationsverbindung zur Steuerung des medizinischen Gerätes zu implementieren. Der integrierte Schaltkreisbaustein kann als Prozessor, Mikroprozessor, Controller oder sonstiger Hardwarebaustein bereitgestellt werden. Dazu kann er einen serverseitigen und einen clientseitigen Bestandteil umfassen. Dementsprechend ist ein erster Abschnitt eines Computerprogramms zur Ausführung des Sicherungsprotokolls auf dem serverseitigen, integrierten Schaltkreisbaustein des Steuerungsgerätes ausgebildet. Die entsprechende, analoge Einheit findet sich als clientseitiger, integrierter Schaltkreisbaustein auf dem medizinischen Gerät wieder, auf dem ein zweiter Abschnitt des Computerprogramms zur Ausführung des Sicherungsprotokolls implementiert ist.

Das Sicherungsprotokoll dient zur eineindeutigen Identifikation des medizinischen Gerätes und zur eineindeutigen Identifikation der jeweiligen Steuerungsinstanz auf dem Steuerungsgerät. Nach korrekter Ausführung des Sicherungsprotokolls kann vorteilhafterweise sichergestellt werden, dass auf dem medizinischen Gerät festgelegt ist, von welchem Steuerungsgerät bzw. von welcher Steuerungsinstanz es gesteuert wird und, dass auf dem Steuerungsgerät umgekehrt festgelegt ist, welches medizinische Gerät gesteuert werden soll. Werden z.B. Steuersignale von anderen Geräten auf dem medizinischen Gerät empfangen, so sind diese unzulässig und es kann gleich ausgeschlossen werden, dass diese zur Steuerung verwendet werden dürfen. Das Sicherungsprotokoll ist vollständig computer-implementiert und wird automatisch ausgeführt.

Fakultativ kann nach dem Aufbau der gesicherten Kommunikationsverbindung bzw. nach der Ausführung des Sicherungsprotokolls ein Verschlüsselungsverfahren aktiviert werden, um die gesicherte Kommunikationsverbindung zusätzlich, also quasi doppelt, zu sichern. Dies ermöglicht es, dass die Annahme "fremder", also nicht zulässiger Steuer- und/oder Statusdatenpakete durch Verschlüsselung sicher ausgeschlossen werden kann. Damit kann die Sicherheit weiterhin verbessert werden.

Identifikationsnachrichten sind elektronische Datenpakete, die zwischen dem medizinischen Gerät und dem Steuerungsgerät ausgetauscht werden. In einer bevorzugten Ausführungsform der Erfindung umfassen diese zumindest eine Initialisierungsnachricht und/oder zumindest eine erste Bestätigungsnachricht und/oder zumindest eine zweite Bestätigungsnachricht.

Alternativ und optional können die Identifikationsnachrichten noch Statusinformationen (z.B. Sensordaten, Gerätezustandsdaten, Versionsnummer von Hardware und/oder Software etc.) umfassen.

Die Initialisierungsnachricht ist eine elektronische Nachricht. Sie dient - als erster Schritt - zur Einleitung der Sicherungsprotokollausführung. Die Initialisierungsnachricht wird auf dem medizinischen Gerät lokal erzeugt. Die Initialisierungsnachricht kann eine eindeutige Geräteidentifikation des medizinischen Gerätes umfassen. Optional können noch weitere Datenfelder in der Initialisierungsnachricht enthalten sein, wie z.B. ein Zeitstempel, Konfigurationsdaten etc. Die Initialisierungsnachricht wird von dem medizinischen Gerät an das oder die Steuerungsgerät(e) gesendet. Das Sicherungsprotokoll kann nur ausgeführt werden, wenn die Initialisierungsnachricht korrekt erzeugt und fehlerfrei auf dem Steuerungsgerät empfangen werden konnte.

Das Triggersignal ist ein elektronisches Signal. Es dient zur Auslösung bzw. zum Triggern der Sicherungsprotokollausführung und ist der Erzeugung der Initialisierungsnachricht vorgeschaltet. Die Initialisierungsnachricht wird in einer bevorzugten Ausführungsform der Erfindung nur erzeugt, wenn das Triggersignal erfasst werden konnte. Das Triggersignal wird lokal auf dem medizinischen Gerät erfasst. Es kann voreingestellt sein, dass das Triggersignal aktiv (z.B. in Form einer Ausgabe auf der Benutzerschnittstelle) angefordert wird. Weiterhin kann voreingestellt sein, dass bei inkorrekter Eingabe oder fehlender Erfassung des Triggersignals, selbiges nochmals wiederholt angefragt wird und/oder ansonsten eine entsprechende Ausgabe erzeugt wird (z.B. im Sinne von "Triggersignal nicht erfasst -> gesicherte Kommunikationsverbindung kann nicht aufgebaut werden"). Das Triggersignal kann die Sicherheit des Verfahrens erhöhen, indem sozusagen eine zweite Sicherheitsstufe (1. Sicherheitsstufe: Erfassen des Triggersignals - 2. Sicherheitsstufe: Erzeugen und Versenden der Initialisierungsnachricht) implementiert wird, die auf Einhaltung überwacht wird. Das Triggersignal kann über die Eingabe eines vordefinierten Benutzersignals erfolgen, das mitunter auf einem Initialisierungsbaustein erfasst wird, der lokal auf dem medizinischen Gerät ausgebildet ist. Der Initialisierungsbaustein kann als Schaltfläche auf einer (z.B. grafischen) Benutzeroberfläche ausgebildet sein oder als Taster und/oder Schalter auf dem medizinischen Gerät. Die Initialisierungsnachricht wird von dem medizinischen Gerät an das Steuerungsgerät gesendet. Darüber hinaus ist es in einer vorteilhaften Variante der Erfindung vorgesehen, dass das Triggersignal nicht explizit durch den Benutzer eingegeben werden muss, sondern es kann indirekt aus einem Gerätezustand automatisch erzeugt und ausgelöst werden. So kann es z.B. vorgesehen sein, dass beim erstmaligen Einschalten des medizinischen Gerätes und/oder im Rahmen einer Inbetriebnahmeprozedur und/oder aufgrund von anderen vorkonfigurierten Ereignissen das Triggersignal automatisch erzeugt wird.

Die auf dem Steuerungsgerät erfassten Steuersignale dienen zur (Fern-)Steuerung des medizinischen Gerätes. Die Steuersignale können automatisch erzeugt sein. Die Steuersignale können auch direkt aus Benutzereingaben eines Anwenders erzeugt werden. Alternativ oder kumulativ können die Steuersignale auch auf Basis der erfassten Benutzereingaben errechnet werden. Die Steuersignale können ein aus einem einzigen Befehl (z.B. Aktiviere/Deaktiviere das medizinische Gerät) oder aus einer Folge von Befehlen bestehen (erste Instruktion, zweite Instruktion... n-te Instruktion, die auf dem medizinischen Gerät auszuführen und anzusteuern ist.

Das Kopplungsergebnis ist eine elektronische Nachricht, die automatisch generiert wird. Das Kopplungsergebnis kann je nach Ausführungsform der Erfindung unterschiedlich ausgebildet sein. Im einfachsten Fall ist es eine Nachricht (sichere Kopplung erfolgreich: Kopplungsnachricht und/oder sichere Kopplung nicht erfolgreich: Fehlkopplungsnachricht). Alternativ können weitere Datenfelder in dem Kopplungsergebnis ausgebildet sein, wie z.B. ein Zeitstempel, der den Beginn des aufgebauten sicheren Kommunikationskanals anzeigt. Alternativ kann der sichere Kommunikationskanal mit einem "Ablaufdatum" ausgebildet sein, so dass der gesicherte Kommunikationskanal nach Ablauf einer vordefinierten Zeitspanne automatisch wieder gelöscht bzw. abgebaut wird. Das Kopplungsergebnis kann darüber hinaus noch weitere Metadaten umfassen. Das Kopplungsergebnis kann mittels eines dafür ausgebildeten Hardwarebausteins ausgegeben werden. Bei dem Hardwarebaustein kann es sich z.B. um eine Ampelschaltung handeln (grünes Signal für erfolgreicher Aufbau und rotes Signal für fehlgeschlagenen Aufbau). Neben oder zusätzlich zu der vorstehend beschriebenen auf einer Farbgebung basierenden Signalisierung kann eine akustische Signalgebung vorgesehen sein. Alternativ oder kumulativ kann ein drittes (z.B. oranges) Signal vorgesehen sein, das signalisiert, dass im Moment gerade das Sicherungsprotokoll zum Aufbau der gesicherten Kommunikationsverbindung ausgeführt wird.

Nachdem der gesicherte Kommunikationskanal aufgebaut worden ist, ist es gemäß einem weiteren Aspekt der Erfindung vorgesehen, dass das Fortbestehen der gesicherten Kommunikationsverbindung kontinuierlich überprüft wird. Damit kann die Sicherheit des verteilten Systems weiterhin erhöht werden. Es wird somit auch sofort erfasst, dass zwar eine gesicherte Kommunikationsverbindung erfolgreich aufgebaut werden konnte, diese aber nach einer gewissen Zeit nicht mehr besteht oder unterbrochen wird (z.B. bei einem vorübergehenden Netzwerkausfall). Im Stand der Technik konnte in solchen Fällen die Sicherheit nicht gewährleistet werden, da das Gerät die Steuersignale in dem Unterbrechungszeitraum nicht ausführen konnte, aber die Folge von nächsten Steuersignalen nach dem Unterbrechungsende jedoch wie geplant ausführt. Somit kann das Fehlen der vorhergehenden Steuersignale nur schwer oder gar nicht erkannt werden. Dies kann in Folge zu einer fehlerhaften Steuerung des medizinischen Gerätes führen. Gemäß einem Aspekt dieser Anmeldung kann dies sicher ausgeschlossen werden, indem das Fortbestehen der (einmal) aufgebauten gesicherten Kommunikationsverbindung kontinuierlich und automatisch überwacht bzw. überprüft wird.

Das Prüfen erfolgt kontinuierlich. Das Prüfen kann nach einem vordefinierten Zeitmuster ausgeführt werden und/oder im Anschluss an den Empfang neuer Telegramme/Datenpakete. So kann in einer Konfigurationsphase definiert werden, dass das Prüfen nach Ablauf eines Zeitintervalls automatisch getriggert wird. Ebenso und/oder kumulativ kann das Prüfen ereignisbasiert getriggert werden (z.B. nach Erfassen eines Eingabesignals auf dem medizinischen Gerät, nach dem Erfassen und vor der Ausführung von bestimmten (z.B. besonders sicherheitsrelevanten) Steuerbefehlen, nach einem Neustart des medizinischen Gerätes und/oder des Steuerungsgerätes oder nach anderen Ereignissen. Das Prüfen erfolgt signalbasiert. Mit anderen Worten wird das Prüfen vorzugsweise automatisch ausgeführt und somit ohne Benutzerinterkation. Damit kann die Sicherheit des Betriebs- und Steuerungsverfahrens erhöht werden. Die Prüfung erfolgt jedoch mindestens innerhalb der zuvor festgelegten Fehlertoleranzzeit.

Das Freigabesignal kann nicht ohne weiteres ein einfaches 1-Bit Signal sein, das über die gesicherte Kommunikationsverbindung an das medizinische Gerät gesendet wird. Das Freigabesignal muss sicher kodiert sein. "Sicher" meint in diesem Zusammenhang, dass eine Verfälschung durch die Signalübertragung und/oder Signalverarbeitung mit hoher Wahrscheinlichkeit nicht zu einer falsch positiven Interpretation durch das Dialysegerät führt. Vorstellbar ist die Verwendung eines Codeworts aus einer großen Zahlenmenge oder bei Verwendung eines 1-Bit Signals der zusätzliche Einsatz von geeigneten Prüfsummen. Dies dient dazu, dass auf dem medizinischen Gerät der Empfang des Freigabesignals überwacht werden kann. Dazu kann im Vorfeld (ebenfalls) ein Protokoll definiert werden, das z.B. festlegt, dass das Freigabesignal in einem bestimmten Zeitintervall und/oder innerhalb einer vordefinierbaren Fehlertoleranzzeit und/oder in einer vordefinierbaren Qualität (z.B. mit korrekter Prüfsumme) empfangen wurde.

Das Sicherungsverfahren und das Betriebsverfahren können als Computerprogramm bereitgestellt werden, insbesondere als Mikroprozessorprogramm.

Beschrieben wird ferner ein Verwenden eines Sicherungsverfahrens zum Aufbau einer gesicherten Kommunikationsverbindung, insbesondere ein Verwenden des Sicherungsverfahrens in dem Steuersystem. Es wird ein Sicherungsprotokoll auf dem Steuerungsgerät und dem medizinischen Gerät ausgeführt zum Herstellen einer gesicherten Kommunikationsverbindung zur Steuerung des medizinischen Gerätes, wobei das Sicherungsprotokoll Identifikationsnachrichten zur eineindeutigen Identifikation des medizinischen Gerätes und zur eineindeutigen Identifikation der jeweiligen Steuerungsinstanz auf dem Steuerungsgerät mit dem medizinischen Gerät austauscht.

Bei dem Verwenden des Sicherungsverfahrens können mehrere Steuerungsinstanzen auf dem Steuerungsgerät ausgebildet sein oder sich mehrere Steuergeräte in einem Netzwerk befinden und bei dem die Initialisierungsnachricht eine Broadcastnachricht an alle Steuerungsinstanzen ist.

Bei dem Verwenden des Sicherungsverfahrens kann die Initialisierungsnachricht einen Ausstattungscode und/oder Kalibrierdaten umfassen, der (jeweils) die technische Ausstattung des medizinischen Gerätes repräsentiert.

Eine weitere Aufgabenlösung besteht somit in einem Computerprogrammprodukt, das in einem Speicher eines Computers oder eines elektronischen oder medizintechnischen Gerätes geladen oder ladbar ist mit einem Computerprogramm zur Durchführung des oben näher beschriebenen Sicherungsverfahrens und/oder des Betriebsverfahrens, wenn das Computerprogramm auf dem Computer oder dem elektronischen oder medizintechnischen Gerät ausgeführt wird.

Eine weitere Aufgabenlösung sieht ein Computerprogramm vor zur Durchführung aller Verfahrensschritte des oben näher beschriebenen Verfahrens, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

Eine weitere Aufgabenlösung sieht ein Datenprotokoll vor, das gemäß dem vorstehend beschriebenen Sicherungsprotokoll ausgebildet ist. Das Datenprotokoll kennzeichnet sich durch den Austausch von Identifikationsnachrichten, die gemäß einer bevorzugten Ausführungsform der Erfindung eine Initialisierungsnachricht und eine erste und zweite Bestätigungsnachricht umfassen.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

Fig. 1 zeigt in einer schematischen Darstellung eine Gruppe von Dialysegeräten, die von einer Steuerungseinheit gemäß einer vorteilhaften Ausführungsform der Erfindung gesteuert werden.
Fig. 2 ist ein Beispiel für ein Ablaufdiagramm für ein Sicherungsverfahren für das Einrichten einer Kommunikationsverbindung zur gesicherten Fernsteuerung eines Dialysegerätes.
Fig. 3 ist ein exemplarisches Interaktionsdiagramm mit einem Datenaustausch von Signalen und Nachrichten zwischen dem Steuerungsgerät mit seinen Steuerungsinstanzen und einem Dialysegerät.
Fig. 4 zeigt eine Darstellung von elektronischen Modulen zum Einrichten einer gesicherten Kommunikationsverbindung zwischen einem Steuerungsgerät und einem Dialysegerät in Form eines Blockdiagramms.
Fig. 5 zeigt ein exemplarisches Interaktionsdiagramm mit einem Datenaustausch von Signalen und Nachrichten für ein Betriebsverfahren für ein Schutzsystem zur fortlaufenden Überwachung des Fortbestehens eines gesicherten Kommunikationskanals und
Fig. 6 ist ein Blockdiagramm mit elektronischen Einheiten eines verteilten Schutzsystems gemäß einer bevorzugten Ausführungsform der Erfindung.

### Detaillierte Beschreibung der Figuren

Im Folgenden wird die Erfindung anhand von Ausführungsbeispielen im Zusammenhang mit den Figuren näher beschrieben.

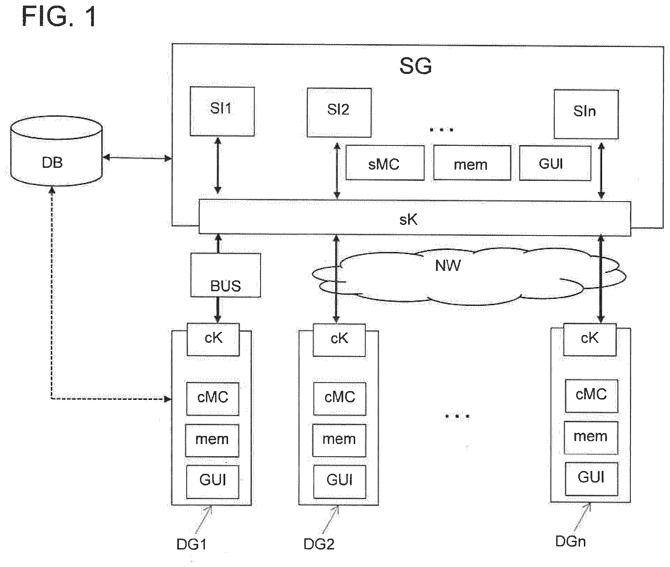

**Fig. 1** zeigt ein Dialysegerät DG als Beispiel für ein medizinisches Gerät, das von einem Steuerungsgerät SG nur dann gesteuert werden soll, wenn eine gesicherte Kommunikationsverbindung zwischen den beiden Geräten aufgebaut worden ist.

Wie in Fig. 1 schematisch dargestellt, kann ein Dialysegerät in einer Gruppe von mehreren Dialysegeräten betrieben werden und zentral von einem Steuerungsgerät gesteuert werden, wobei letzteres z.B. als Steuerserver ausgebildet sein kann. Dazu sind auf dem als Steuerungsgerät SG fungierenden Server mehrere Steuerungsinstanzen SI ausgebildet, die jeweils genau einem zu steuerndem Dialysegerät DG zugeordnet sind. In Fig.1 sind exemplarisch 1 bis n Steuerungsinstanzen SI für 1 bis n Dialysegeräte DG vorgesehen. Die Dialysegeräte DG können über unterschiedliche Netzwerke mit dem Steuerungsgerät SG verbunden sein, wie z.B. über ein Bussystem oder über ein drahtloses Netzwerk NW zum digitalen Datenaustausch.

Zum Aufbau einer gesicherten Kommunikationsverbindung umfasst das Dialysegerät DG eine clientseitige Kommunikationseinrichtung cK, die dazu bestimmt ist, zu diesem Zweck mit einer serverseitigen Kommunikationseinrichtung sK zu kommunizieren. Weiterhin kann auf dem Dialysegerät DG ein clientseitiger integrierter Schaltkreisbaustein cMC ausgebildet sein, sowie ein Speicher mem und eine Benutzerschnittstelle GUI, die z.B. grafisch sein kann.

Das Steuerungsgerät SG umfasst mehrere Steuerungsinstanzen SI, wobei eine erste Steuerungsinstanz SI1 dem ersten Dialysegerät DG1, eine zweite Steuerungsinstanz SI2 dem zweiten Dialysegerät DG2 etc. zugeordnet ist. Zur Kommunikation umfasst das Steuerungsgerät SG die serverseitige Kommunikationseinrichtung. Darüber hinaus ist auf dem Steuerungsgerät SG der serverseitige integrierte Schaltkreisbaustein sMC und optional ein Speicher mem sowie optional eine Benutzeroberfläche GUI ausgebildet.

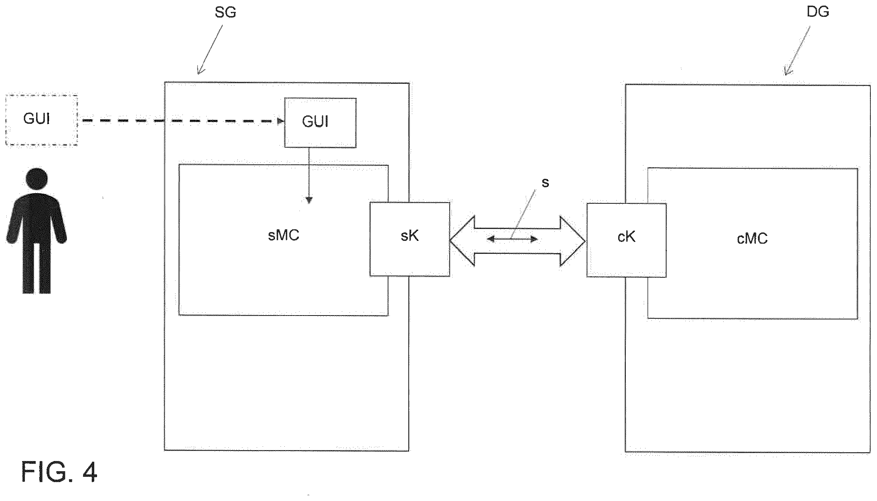

Die Benutzeroberfläche kann in einer alternativen Ausführungsform der Erfindung kumulativ oder alternativ auf einem externen Gerät ausgebildet sei, das datentechnisch mit dem Steuerungsgerät SG gekoppelt ist. Dies ist in Figur 4 dadurch gekennzeichnet, dass die Benutzerschnittstelle GUI in gestrichelter Form auch separat und extern des Steuerungsgerätes zur Verfügung gestellt werden kann. Dann werden vorzugsweise zusätzliche Absicherungsverfahren eingesetzt, z.B. über die Positionsortung des mobilen Schnittstellengerätes, das als Benutzeroberfläche GUI fungierten soll.

Das Steuerungsgerät SG kann - wie in Fig. 1 angedeutet - mit einer Datenbank DB in Datenaustausch stehen. Dort können z.B. Regeln zur Ausbildung des Sicherungsprotokolls ausgebildet und hinterlegt sein, die auch unabhängig vom Dialysegerät DG und vom Steuerungsgerät SG geändert werden können. Optional kann das Dialysegerät oder ausgewählte oder alle Dialysegeräte mit der Datenbank DB verbunden sein, um z.B. auf Konfigurationen und Regeln zugreifen zu können.

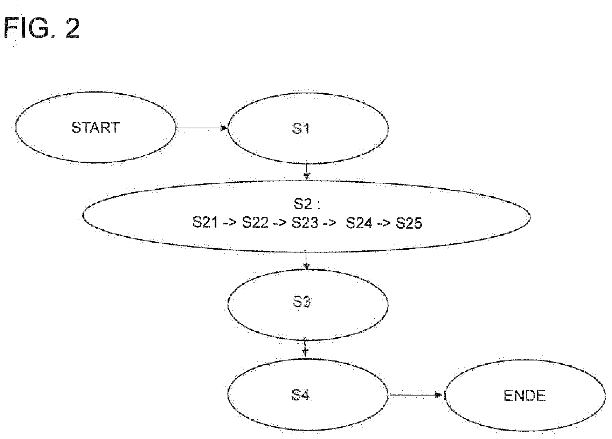

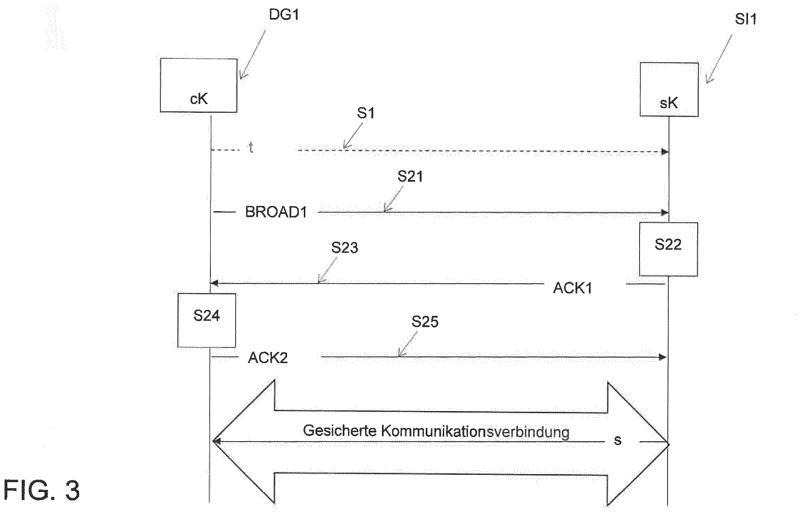

Der Aufbau der gesicherten Verbindung wird lokal von dem Dialysegerät DG aus getriggert und/oder initiiert. Dazu kann (optional) - wie in **Fig. 2** und **Fig. 3** dargestellt - in einem ersten Schritt S1 ein Triggersignal t auf dem Dialysegerät DG erfasst werden, um den Aufbau der gesicherten Kommunikationsverbindung und die Ausführung des Sicherungsprotokolls in Schritt S2 zu triggern. Das Ausführen des Sicherungsprotokolls S2 umfasst vorzugsweise folgende Schritte:
- Senden S21 einer Initialisierungsnachricht BROADC1 mit einer eindeutigen Geräteidentifikation für das medizinische Gerät DG von dem medizinischen Gerät DG an das Steuerungsgerät SG;
- Empfangen S22 der Initialisierungsnachricht BROADC1 auf dem Steuerungsgerät SG;
- Seitens des Steuerungsgerätes SG: Senden S23 einer ersten Bestätigungsnachricht ACK1 an das medizinische Gerät DG mit einer Identifikation der zugeordneten Steuerungsinstanz in Antwort auf die Initialisierungsnachricht;
- Empfangen S24 der ersten Bestätigungsnachricht ACK1 auf dem medizinischen Gerät DG und
- Seitens des medizinischen Gerätes DG: Senden S25 einer zweiten Bestätigungsnachricht ACK2 in Antwort auf die empfangene erste Bestätigungsnachricht ACK1 an das Steuerungsgerät SG.

Optional kann während oder vor Schritt S22 eine Konfiguration der jeweiligen Steuerungsinstanz SI ausgeführt werden. Insbesondere erfolgt jedoch nach Schritt S22 eine Konfiguration auf Basis der mit der Initialisierungsnachricht BROADC1 empfangenen Daten. Dies ist mitunter dann hilfreich, wenn die Initialisierungsnachricht BROADC1 Konfigurationsdaten des medizinischen Gerätes DG enthält (z.B. Versionsnummer, Ausstattungscode und dergleichen). Des Weiteren kann so ein Automatismus implementiert werden, der im Bedarfsfall automatisch neue, benötigte Serverinstanzen SI generiert bzw. anlegt.

**Fig. 3** zeigt ein Interaktionsdiagramm zwischen der clientseitigen Kommunikationseinrichtung cK auf dem Dialysegerät DG und der serverseitigen Kommunikationseinrichtung sK auf dem Steuerungsgerät SG. Zunächst wird das Triggersignal t auf dem Dialysegerät DG erfasst und dann an das Steuerungsgerät SG gesendet. Das Triggersignal wird benötigt, um das Dialysegerät DG überhaupt erst in den Pairing-Modus zu versetzen. Ohne dieses Triggersignal (z.B. kann eine vordefinierte Prozedur am Dialysegerät DG, z.B. eine Erst-Inbetriebnahmeprozedur als Triggersignal fungieren) muss das Gerät DG verhindern, dass der Pairing-Modus gestartet wird. Dies ist eine Schutzfunktionalität. Vor allem wird so sichergestellt, dass nicht während der Behandlung der Pairing-Modus aktiv werden kann. Dieser Schritt kann zeitlich von den nachfolgenden Schritten entkoppelt sein und ist deshalb in Fig. 3 gestrichelt dargestellt. Anschließend wird die Initialisierungsnachricht BROADC1 an das Steuerungsgerät SG gesendet, das in Antwort darauf die erste Bestätigungsnachricht ACK1 an das Dialysegerät DG sendet, was dort mit dem Versenden der zweiten Bestätigungsnachricht ACK2 (vorzugsweise als Broadcast Nachricht versendet) beantwortet wird. Erst nach diesem vordefinierten Handshake-Protokoll wird die gesicherte Kommunikationsverbindung zwischen den beiden Instanzen aufgebaut.

Sollten mehrere Steuergeräte SG zeitgleich versucht haben, sich beim Dialysegerät DG zum Zwecke des Aufbaus einer gesicherten Kommunikationsverbindung zur Steuerung des Gerätes DG zu registrieren, dann wird durch das Empfangen der zweiten Bestätigungsnachricht ACK2 eineindeutig das Steuerungsgerät SG / derjenige Server bzw. diejenige Serverinstanz SI identifiziert, die zur Steuerung bestimmt worden ist. Die anderen Geräte/Instanzen, die keine zweite Bestätigungsnachricht ACK2 innerhalb einer vordefinierbaren Zeitspanne erhalten haben, welche sie als akzeptierte Serverinstanz SI ausweist, sind nicht sicher gekoppelt und können dies über eine entsprechende Meldung signalisieren. Optional kann das sicher gekoppelte Steuerungsgerät SG eine entsprechende Meldung über das Kopplungsergebnis ausgeben.

**Fig. 4** zeigt schematisch nochmals überblicksartig die strukturelle Architektur der erfindungsgemäßen Lösung. Der Anwender tätigt seine Eingaben zur Steuerung des Dialysegerätes DG auf dem entfernten Steuerungsgerät SG. Um sicherzustellen, dass der Kommunikationskanal zwischen den beiden Instanzen auch gesichert ist, wird das Sicherungsprotokoll in den beiden integrierten Schaltkreisbausteinen cMC, sMC gespeichert und ausgeführt. Erst wenn die korrekte Ausführung des Sicherungsprotokolls durchgeführt worden ist und die gesicherte Kommunikationsverbindung hergestellt worden ist, können Steuerbefehle s von dem Steuerungsgerät SG an das Dialysegerät DG übertagen werden.

In einer alternativen vorteilhaften Ausführungsform der Erfindung kann - wie in Fig. 4 gestrichelt dargestellt - die Benutzerschnittstelle GUI auch außerhalb des Steuerungsgerätes SG angeordnet sein, etwa auf einem mobilen Endgerät des Anwenders, das über drahtlose Kommunikationsverbindung mit dem Steuerungsgerät SG verbunden ist. Damit kann das Steuerungsverfahren noch flexibler gestaltet werden, während gleichzeitig den erforderlichen Sicherheitsanforderungen genüge getan ist. Dabei kann die Benutzerschnittstelle GUI Teil von weiteren Automatismen und Interaktionen mit dem Dialysegerät DG sein. Bspw. kann eine Näherung eines mobilen Endgerätes zum Dialysegerät DG automatisch erkannt werden (siehe: EP2925381B1), woraufhin die Benutzerschnittstelle GUI vom Steuerungsgerät SG oder vom Dialysegerät DG automatisch die aktuellen Behandlungsdaten und Eingabemasken abfragen und auf der Benutzerschnittstelle GUI anzeigen kann. (Funkbasierte Näherungserkennung/Ortung bspw. via Bluetooth "Google Eddystone" / "Apple iBeacon"). Im Unterschied dazu, ist der Kommunikationsablauf allerdings leicht geändert. Nachdem das GUI den "Beacon" empfangen hat, der auch die Dialysegeräte-Identifikation DG-ID enthält, kommuniziert die Benutzerschnittstelle GUI mit dem Steuerungsgerät SG und bekommt von diesem die Daten bzw. sendet Eingaben an das Steuerungsgerät SG statt direkt an das Dialysegerät DG. Dies ist schematisch auch in Fig. 4 dargestellt. Grundsätzlich ist es auch möglich, dass das mobile Endgerät (z.B. Tablet) seine Identifikationsdaten an das medizinische Gerät DG übertragt und dieses dann diese Identifikationsdaten an das Steuerungsgerät SG weiterleitet. Das Steuerungsgerät SG sendet dann die jeweiligen Inhalte direkt an das mobile Gerät.

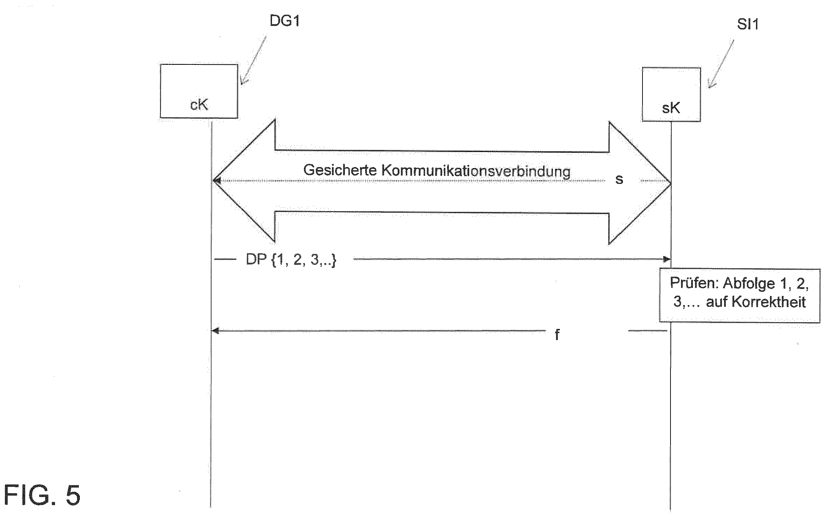

Unter Bezugnahme auf **Fig. 5** wird ein Betriebsverfahren für ein verteiltes Schutzsystem SchS näher erläutert. Dazu wird im Vorfeld eine gesicherte Kommunikationsverbindung nach einem der vorher beschriebenen Verfahren aufgebaut (Sicherungsverfahren). Das Schutzsystem bzw. das Betriebsverfahren dienen nun dazu, kontinuierlich zu prüfen, ob die aufgebaute gesicherte Kommunikationsverbindung auch weiterhin zuverlässig besteht.

Das verteilte Schutzsystem SchS umfasst ein Client-Schutzsystem C-SchS, das auf dem Dialysegerät DG implementiert ist und ein Server-Schutzsystem S-SchS, das auf einem entfernten Steuerungsgerät SG implementiert sein kann, und bei dem kontinuierlich überprüft wird, ob eine gesicherte Kommunikationsverbindung zwischen dem medizinischen Gerät DG und dem Steuerungsgerät SG fortbesteht (erster Aspekt der Prüfung) und, ob der Behandlungsablauf korrekt ist (zweiter Aspekt der Prüfung), zum Beispiel durch Vergleich mit einem vordefinierbaren Referenzablauf, der definiert, dass kein Patientengefährdungszustand besteht oder durch Überwachung kritischer Parameter, wie z.B. der Bluttemperatur, der Leitfähigkeit und damit indirekt der Zusammensetzung des Dialysats, Luftfreiheit im extrakorporalen Blutkreislauf. Das Betriebsverfahren kann dazu folgende, automatisch ausgeführte Schritte ausführen:
Auf dem Server-Schutzsystem S-SchS erfolgt ein signalaustausch-basiertes Prüfen, ob eine gesicherte Kommunikationsverbindung zwischen dem medizinischen Gerät DG und dem Steuerungsgerät SG fortbesteht und falls JA wird zumindest eines Freigabesignals f zyklisch an das medizinische Gerät DG gesendet. Falls z.B. zwischen dem Sendezeitpunkt A und dem darauffolgenden Sendezeitpunkt B für das Freigabesignal erkannt wird, dass das medizinischen Gerät DG keine, falsche oder zu wenige Daten an das Steuerungsgerät SG übertragen hat, unterbleibt das Versenden des Freigabesignals zum Zeitpunkt B.

Seitens des Client-Schutzsystem C-SchS erfolgt ein vollautomatisches Überwachen, ob das zumindest eine Freigabesignal f innerhalb einer vordefinierten Fehlertoleranzzeit empfangen wurde (und somit als korrektes Freigabesignal gewertet werden kann) und falls NEIN wird das medizinische Gerät DG automatisch in einen sicheren Gerätezustand überführt, der die Patientensicherheit nicht gefährdet. Dies hat den Vorteil, dass mitunter Fehler im Steuerungsgerät SG und/oder ein Verlust oder eine Unterbrechung der Datenverbindung zwischen Steuerungsgerät SG und Dialysegerät DG automatisch detektiert werden können, um die sicherheitsrelevanten Maßnahmen automatisch einleiten zu können. Sinn und Zweck des Server-Schutzsystems S-SchS ist, dass die jeweilige Schutzsysteminstanz auf dem Steuerungsgerät SG (bzw. die Steuerungsinstanz SI) die Steuersignale des Betriebssystems, das ebenfalls Teil des Steuerungsgerätes SG ist, überwacht und nur bei korrekten Steuersignalen ein weiteres Freigabesignal an das Dialysegerät DG sendet, um somit die vorherigen Daten vom Betriebssystemteil von Steuerungsgerät SG/Steuerungsinstanz SI zu bestätigen.

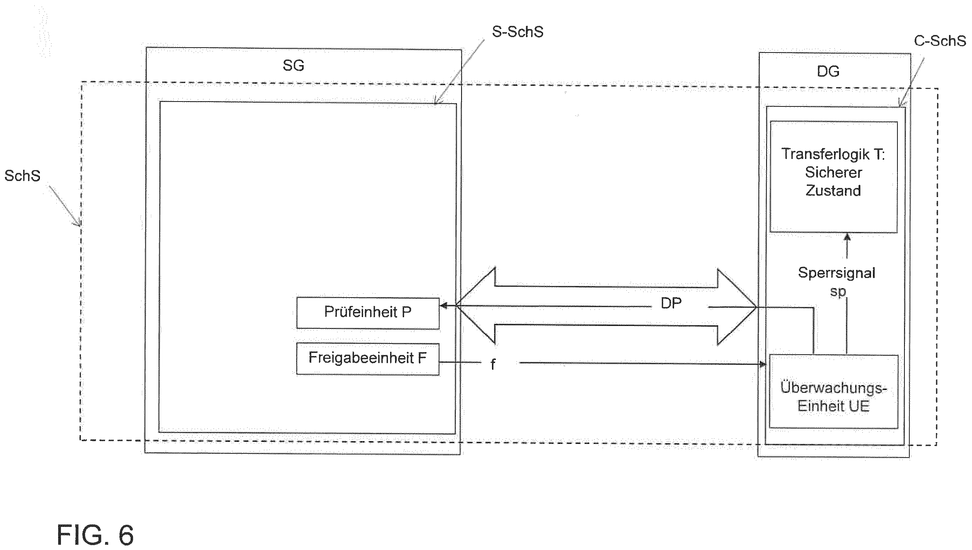

In **Fig. 6** ist ein Blockdiagramm mit Modulen des verteilten Schutzsystem SchS gemäß einem Ausführungsbeispiel gezeigt. Da das Schutzsystem SchS auf mehreren physikalischen Produkten SG, DG verteilt realisiert ist, ist es in Fig. 6 gestrichelt dargestellt. Das Server-Schutzsystem S-SchS umfasst die Prüfeinheit P und die Freigabeeinheit F, während das Client-Schutzsystem C-SchS die Transferlogik T und die Überwachungseinheit UE umfasst.

Nach dem Aufbau der gesicherten Kommunikationsverbindung durch das vorher beschriebene Sicherungsverfahren, wird über diese Verbindung kontinuierlich das Versenden von Status-Datenpaketen DP seitens des Dialysegerätes veranlasst. Diese Datenpakete DP können vorteilhafterweise einen Zeitstempel, eine Sequenznummer und/oder Sensorsignale enthalten. Die Prüfeinheit P prüft daraufhin auf dem Server-Schutzsystem S-SchS, ob die Datenpakete in der konsekutiv korrekten Sequenz empfangen worden sind. Dazu kann entweder eine SOLL-Sequenz vorgegeben sein oder eine Referenz-Sequenz wird in einer zentralen Datenbank abgelegt und für den Vergleich verwendet. Bei erfolgreicher Prüfung (korrekter Empfang alleine genügt nicht) wird das Freigabesignal f an das Client-Schutzsystem C-SchS gesendet. Die dortige Überwachungseinheit UE dient nun dazu, fortlaufend zu überwachen, ob ein Freigabesignal f erfasst werden kann. Dazu kann ein vorbestimmtes Prüfschema hinterlegt sein (z.B. zeitbasiert, etwa alle 5 min und/oder ereignisbasiert, etwa nach dem Eintritt vorbestimmbarer Ereignisse). Falls ja, werden weiter Datenpakete DP gesendet und der Betrieb kann aufrechterhalten werden, da sichergestellt ist, dass die gesicherte Kommunikationsverbindung fortbesteht und der Behandlungsablauf korrekt, d.h. ohne Patientengefährdung durchgeführt wird. Andernfalls wird sofort ein Sperrsignal sp erzeugt, das an die interne Transferlogik T weitergegeben wird, um das Dialysegerät DG in einen sicheren Zustand zu überführen. Anstelle des Sperrsignals sp kann auch ein weiteres Freigabesignal vorgesehen sein, das dann nicht gesendet wird, wenn ansonsten das Sperrsignal sp gesendet werden würde. Das weitere Freigabesignal wird somit gesendet, wenn die gesicherte Kommunikationsverbindung fortbesteht und der Behandlungsablauf korrekt ist (kein Patientengefährdungszustand besteht). Das weitere Freigabesignal wird nicht gesendet, wenn keine gesicherte Kommunikationsverbindung fortbesteht oder wenn der Behandlungsablauf nicht korrekt ist (bzw. ein Patientengefährdungszustand besteht). Optional kann zusätzlich eine Ausgabe auf einer Benutzerschnittstelle des Dialysegerätes DG und/oder des Steuerungsgerätes SG erfolgen (z.B. als Warnhinweis). Die Ausgabe kann auf einem Hardwarebaustein erfolgen, der in Form einer Ampel ausgebildet sein kann mit entsprechender Signalgebung: ROT für "Unterbrechung der gesicherten Kommunikationsverbindung + Transfer in sicheren Gerätezustand" und GRÜN für "Fortbestehen der gesicherten Kommunikationsverbindung + weiteres Betreiben des Dialysegerätes im abgesicherten Fernsteuermodus".

Wichtig ist, dass das Dialysegerät DG vom Steuerungsgerät SG gesteuert wird, genauer gesagt von einer Steuerungsinstanz SI auf dem Steuerungsgerät SG bzw. einer Betriebssysteminstanz. Diese sendet Steuersignale an das Dialysegerät DG (z.B. zum Steuern von Aktorik). Zuvor und/oder danach hat das Dialysegerät DG Datenpakete (Sensorsignale, Statusinfos zum Dialysegerät DG etc.) an die Steuerungsinstanz SI übermittelt. Dies alles erfolgt innerhalb eines "Slices"/Zeitabschnitts. Wenn die Steuerungsinstanz SI anhand der Prüfung der Datenpakete und ggfs. der Steuersignale von der Steuerungsinstanz zu dem Schluss kommt, dass keine Patientengefährdung vorliegt, versendet die Steuerungsinstanz SI das weitere Freigabesignal an das Dialysegerät DG. Bspw. kann die Steuerungsinstanz SI die Bluttemperatur aus den Datenpaketen vom Dialysegerät DG auswerten, um festzustellen, dass diese über dem zulässigen Wert von z.B. 41°C liegt, so dass die Steuerungsinstanz SI kein Freigabesignal versendet. Oder: Die Steuerungsinstanz SI stellt fest, dass eine Betriebssysteminstanz seine Abarbeitungsreihenfolge nicht einhält und die Steuersignale teilweise fehlen oder in der falschen Reihenfolge an das Gerät DG übermittelt werden, so dass ebenfalls kein weiteres Freigabesignal versendet wird. Generell ist es möglich, dass das Schutzsystem, welches den zweiten Abschnitt der Prüfung übernimmt (Verfahrensablauf korrekt, z.B. Bluttemperatur unterhalb des Grenzwertes) im Dialysegerät DG verbleibt und nur das Betriebssystem im externen Steuerungsgerät SG realisiert wird. Damit kann ein höheres Maß an Sicherheit gewährleistet werden.

Zusammenfassend stellt die vorliegende Anmeldung ein Schutzkonzept bereit mit einem Sicherungsverfahren und einem Betriebsverfahren und entsprechenden elektronischen Einheiten, um eine abgesicherte Fernbedienung eines medizinischen Gerätes zu ermöglichen. Dabei wird die Absicherung fortlaufend überprüft, um das zu steuernde Gerät automatisch in einen sicheren Zustand zu bringen, sobald erfasst wird, dass die gesicherte Kommunikationsverbindung nicht mehr besteht oder vorübergehend oder vollständig unterbrochen worden ist oder fehlerhaft ist. Durch die Ausführung des Sicherungsprotokolls als Handshake-Protokoll ist die Kommunikationsverbindung vorteilhafterweise mehrfach abgesichert.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung neben dem oder anstelle des Netzwerkes NW zwischen dem Dialysegerät DG und dem Steuerungsgerät SG andere Schnittstellen oder Verbindungen vorzusehen. Für einen Fachmann ist es insbesondere offensichtlich, dass die Erfindung nicht nur für Dialysegeräte (z.B. für ein Hämodialysegerät oder ein Peritonealdialysegerät) angewendet werden kann, sondern auch für andere medizinische Geräte, die in abgesicherter Weise über ein entferntes Steuerungsgerät SG gesteuert werden müssen.

Des Weiteren können die Bauteile des medizinischen Gerätes DG und des Steuerungsgerätes SG, wie die Benutzerschnittstelle GUI etc. auf mehrere physikalische Produkte verteilt realisiert werden.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. **Steuerungssystem** zum Aufbau einer gesicherten Kommunikationsverbindung zum Steuern von zumindest einem medizinischen Gerät (DG), insbesondere einem Dialysegerät, mit einem Steuerungsgerät (SG), das entfernt von dem medizinischem Gerät (DG) angeordnet ist, mit folgenden Einheiten:
dem zumindest einen **medizinischen Gerät (DG)**, das über das entfernte Steuerungsgerät (SG) gesteuert wird, mit
- einer clientseitigen Kommunikationsreinrichtung (cK), die zum Datenaustausch mit einer serverseitigen Kommunikationseinrichtung (sK) des Steuerungsgerätes (SG) ausgebildet ist, und
- einem integrierten Schaltkreisbaustein (cMC), der ausgebildet ist, ein Sicherungsprotokoll zum Herstellen einer gesicherten Kommunikationsverbindung zu dem Steuerungsgerät (SG) zu implementieren, und wobei das Sicherungsprotokoll Identifikationsnachrichten zur eineindeutigen Identifikation des medizinischen Gerätes (DG) und zur eineindeutigen Identifikation einer jeweiligen Steuerungsinstanz (SI) auf dem Steuerungsgerät (SG) austauscht, wobei jeweils eine Steuerungsinstanz (SI) zur dedizierten Steuerung eines bestimmten medizinischen Gerätes (DG) bestimmt ist;
dem **Steuerungsgerät (SG)**, umfassend zumindest eine Steuerungsinstanz (SI), die jeweils zur Steuerung zumindest eines medizinischen Gerätes (DG) beauftragt ist, mit
- einer serverseitigen Kommunikationseinrichtung (sK) zur Kommunikation mit dem medizinischen Gerät (DG); und
- einem integrierten Schaltkreisbaustein (cMC), der ausgebildet ist, ein Sicherungsprotokoll zum Herstellen einer gesicherten Kommunikationsverbindung zur Steuerung des medizinischen Gerätes (DG) zu implementieren, und wobei das Sicherungsprotokoll Identifikationsnachrichten zur eineindeutigen Identifikation des medizinischen Gerätes (DG) und zur eineindeutigen Identifikation der jeweiligen Steuerungsinstanz (SI) auf dem Steuerungsgerät (SG) zwischen dem medizinischen Gerät (DG) und dem Steuerungsgerät (SG) austauscht,
wobei das Steuerungsgerät (SG) eine **Benutzerschnittstelle (GUI)** aufweist, zum Erfassen von Steuersignalen (s) zur Steuerung des medizinischen Geräts (DG) und/oder zur Ausgabe von Zustandsdaten des medizinischen Geräts (DG), wobei die **Benutzerschnittstelle (GUI) auf einem separaten Gerät außerhalb des Steuerungsgerätes (SG) ausgebildet ist**, das mit dem Steuerungsgerät (SG) in bidirektionalem Datenaustausch steht, wobei die Benutzerschnittstelle (GUI) nur dann - insbesondere über das medizinische Gerät - mit dem entfernten Steuerungsgerät (SG) kommunizieren kann, wenn ein **Positionssensor erkennt, dass sich die Benutzerschnittstelle (GUI) in einer räumlichen Nähe zum medizinischen Gerät** befindet; und
wobei alle der Einheiten über zumindest ein Netzwerk (NW) im Datenaustausch stehen.

2. Steuerungssystem nach Anspruch 1, wobei das Steuerungsgerät (SG) zur Ausführung des Sicherungsprotokolls durch Empfangen einer Initialisierungsnachricht (BROADC1) auf dem Steuerungsgerät (SG) aktiviert wird.

3. Sicherungsverfahren zum Aufbau einer gesicherten Kommunikationsverbindung zwischen zumindest einem medizinischen Gerät (DG), insbesondere einem Dialysegerät und einem entfernten Steuerungsgerät (SG) zur Verwendung in einem Steuerungssystem nach Anspruch 1 oder 2, wobei das Steuerungsgerät (SG) zumindest eine Steuerungsinstanz (SI) umfasst, die jeweils zur Steuerung zumindest eines medizinischen Gerätes (DG) beauftragt ist, mit folgenden Verfahrensschritten:
- Ausführen (S2) eines Sicherungsprotokolls auf dem Steuerungsgerät (DG) und dem medizinischen Gerät (DG) zum Herstellen einer gesicherten Kommunikationsverbindung zur Steuerung des medizinischen Gerätes (DG), wobei das Sicherungsprotokoll Identifikationsnachrichten zur eineindeutigen Identifikation des medizinischen Gerätes (DG) und zur eineindeutigen Identifikation der jeweiligen Steuerungsinstanz (SI) auf dem Steuerungsgerät (SG) mit dem medizinischen Gerät (DG) austauscht.

4. Sicherungsverfahren nach Anspruch 3, mit folgendem Schritt:
- Erfassen (S1) eines Triggersignals (t) auf dem medizinischen Gerät (DG), um die Ausführung des Sicherungsprotokolls auszulösen.

5. Sicherungsverfahren nach Anspruch 3 oder 4, mit folgenden Schritten:
- Senden (S21) einer Initialisierungsnachricht (BROADC1) mit einer eindeutigen Geräteidentifikation für das medizinische Gerät (DG) von dem medizinischen Gerät (DG) an das Steuerungsgerät (SG);
- Empfangen (S22) der Initialisierungsnachricht (BROADC1) auf dem Steuerungsgerät (SG), wobei die Initialisierungsnachricht (BROADC1) einen Ausstattungscode und/oder Kalibrierdaten umfasst, der die technische Ausstattung des medizinischen Gerätes (DG) repräsentiert;
- seitens des Steuerungsgerätes (SG): Senden (S23) einer ersten Bestätigungsnachricht (ACK1) an das medizinische Gerät (DG) mit einer Identifikation der zugeordneten Steuerungsinstanz (SI) in Antwort auf die Initialisierungsnachricht (BROADC1);
- Empfangen (S24) der ersten Bestätigungsnachricht (ACK1) auf dem medizinischen Gerät (DG); und
- seitens des medizinischen Gerätes (DG): Senden (S25) einer zweiten Bestätigungsnachricht (ACK2) in Antwort auf die empfangene erste Bestätigungsnachricht (ACK1) an das Steuerungsgerät (SG).

6. Sicherungsverfahren gemäß Anspruch 5, bei dem das Steuerungsgerät (SG) nach dem Empfangen der Initialisierungsnachricht (BROADC1) in Antwort auf die empfangene Initialisierungsnachricht (BROADC1) konfiguriert wird.

7. Sicherungsverfahren nach Anspruch 5 oder 6, bei dem mehrere Steuerungsinstanzen (SI) auf dem Steuerungsgerät (SG) ausgebildet sind oder sich mehrere Steuergeräte (SG) in einem Netzwerk befinden und bei dem die Initialisierungsnachricht (BROADC1) eine Broadcastnachricht an alle Steuerungsinstanzen (SI) ist.

8. Sicherungsverfahren nach einem der Ansprüche 5 bis 7, bei dem die Initialisierungsnachricht (BROADC1) einen Ausstattungscode und/oder Kalibrierdaten umfasst, der die technische Ausstattung des medizinischen Gerätes (DG) repräsentiert.

9. Sicherungsverfahren nach einem der Ansprüche 5 bis 8, bei dem nach Ausführung des Sicherungsprotokolls ein Kopplungsergebnis auf einer Benutzerschnittstelle des Steuerungsgerätes (SG) und/oder des medizinischen Gerätes (DG) angezeigt wird, oder das Kopplungsergebnis wird mittels einer Aktivierung eines Hardwarebausteins signalisiert.

10. Sicherungsverfahren nach einem der Ansprüche 5 bis 9, bei dem die Ausführung des Sicherungsprotokolls umfasst:
- Seitens des Steuerungsgerätes (SG): Auswerten, ob eine zweite Bestätigungsnachricht (ACK2) in Antwort auf eine empfangene erste Bestätigungsnachricht (ACK1) auf dem Steuerungsgerät (SG) innerhalb einer vordefinierten Timeout-Zeitspanne empfangen wurde, und verneinendenfalls: Ausgeben einer Fehlkopplungsnachricht auf dem Steuerungsgerät (SG) und/oder bejahendenfalls: Ausgaben einer Kopplungsnachricht auf dem Steuerungsgerät (SG).

## Claims

1. **Control system** for establishing a secure communication link for controlling at least one medical device (DG), in particular a dialysis machine, with a control device (SG) remote from the medical device (DG), comprising the following units:
the at least one **medical device (DG)**, which is controlled via the remote control device (SG), with
- a client-side communication device (cK), which is confirmed to exchange data with a server-side communication device (sK) of the control device (SG), and
- an integrated circuit module (cMC) which is confirmed to implement a security protocol for establishing a secure communication connection to the control device (SG), and wherein the security protocol exchanges identification messages for uniquely identifying the medical device (DG) and for uniquely identifying a respective control instance (SI) on the control device (SG), wherein a respective control instance (SI) is intended for the dedicated control of a specific medical device (DG);
the **control device (SG)**, comprising at least one control instance (SI), each assigned to control at least one medical device (DG), with
- a server-side communication device (sK) for communicating with the medical device (DG); and
- an integrated circuit module (cMC) which is confirmed to implement a security protocol for establishing a secure communication link for controlling the medical device (DG), and wherein the security protocol exchanges identification messages for uniquely identifying the medical device (DG) and for uniquely identifying the respective control instance (SI) on the control device (SG) between the medical device (DG) and the control device (SG),
wherein the control device (SG) has a **user interface (GUI)** for detecting control signals (s) for controlling the medical device (DG) and/or for outputting status data of the medical device (DG), wherein the **user interface (GUI) is formed on a separate device outside the control device (SG)**, which is in bidirectional data exchange with the control device (SG), wherein the user interface (GUI) can only communicate with the remote control device (SG) - in particular via the medical device - if a **position sensor detects that the user interface (GUI)** is **in a spatial proximity to the medical device**; and
whereby all of the units exchange data at least one network (NW).

2. Control system according to claim 1, wherein the control device (SG) is activated to execute the security protocol by receiving an initialization message (BROADC1) on the control device (SG).

3. Security method for establishing a secure communication link between at least one medical device (DG), in particular a dialysis machine, and a remote control device (SG) for use in a control system according to claim 1 or 2, wherein the control device (SG) comprises at least one control instance (SI), each of which is assigned to control at least one medical device (DG), comprising the following method steps
- execution (S2) of a security protocol on the control device (DG) and the medical device (DG) for establishing a secure communication link for controlling the medical device (DG), wherein the security protocol exchanges identification messages for uniquely identifying the medical device (DG) and for uniquely identifying the respective control instance (SI) on the control device (SG) with the medical device (DG).

4. Securing method according to claim 3, comprising the following step:
- Detection (S1) of a trigger signal (t) on the medical device (DG) to initiate the execution of the security protocol.

5. Securing method according to claim 3 or 4, comprising the following steps:
- sending (S21) an initialization message (BROADC1) with a unique device identification for the medical device (DG) from the medical device (DG) to the control device (SG);
- receiving (S22) the initialization message (BROADC1) on the control device (SG), wherein the initialization message (BROADC1) comprises an equipment code and/or calibration data representing the technical equipment of the medical device (DG)
- on the part of the control device (SG): Sending (S23) of a first confirmation message (ACK1) to the medical device (DG) with an identification of the assigned control instance (SI) in response to the initialization message (BROADC1);
- receiving (S24) the first acknowledgement message (ACK1) on the medical device (DG); and
- on the part of the medical device (DG): Sending (S25) a second confirmation message (ACK2) to the control device (SG) in response to the received first confirmation message (ACK1).

6. Security method according to claim 5, wherein the control device (SG) is configured after receiving the initialization message (BROADC1) in response to the received initialization message (BROADC1).

7. Security method according to claim 5 or 6, wherein several control instances (SI) are formed on the control device (SG) or several control devices (SG) are located in a network and wherein the initialization message (BROADC1) is a broadcast message to all control instances (SI).

8. Security method according to any one of claims 5 to 7, wherein the initialization message (BROADC1) comprises an equipment code and/or calibration data representing the technical equipment of the medical device (DG).

9. Security method according to any one of claims 5 to 8, wherein after execution of the security protocol, a pairing result is displayed on a user interface of the control device (SG) and/or the medical device (DG), or the pairing result is signaled by means of an activation of a hardware component.

10. Security method according to any one of claims 5 to 9, wherein executing the security protocol comprises:
- on the part of the control device (SG): evaluating whether a second acknowledgement message (ACK2) was received in response to a received first acknowledgement message (ACK1) on the control device (SG) within a predefined timeout period, and if negative: outputting a miscoupling message on the control device (SG) and/or if affirmative: outputting a coupling message on the control device (SG).

## Revendications

1. **Système de commande** pour l'établissement d'une liaison de communication sécurisée pour la commande d'au moins un appareil médical (DG), en particulier un appareil de dialyse, avec un appareil de commande (SG) qui est disposé à distance de l'appareil médical (DG), avec les unités suivantes :
l'au moins un **appareil médical (DG) qui** est commandé par l'intermédiaire du dispositif de commande distant (SG), avec
- un dispositif de communication (cK) côté client, qui est conçu pour l'échange de données avec un dispositif de communication (sK) côté serveur de l'appareil de commande (SG), et
- un module de circuit intégré (cMC) qui est conçu pour mettre en oeuvre un protocole de sécurité pour établir une liaison de communication sécurisée avec l'appareil de commande (SG), et le protocole de sécurité échangeant des messages d'identification pour l'identification univoque de l'appareil médical (DG) et pour l'identification univoque d'une instance de commande (SI) respective sur l'appareil de commande (SG), une instance de commande (SI) étant respectivement destinée à la commande dédiée d'un appareil médical (DG) déterminé ;
le **dispositif de commande (SG)**, comprenant au moins une instance de commande (SI), dont chacune est chargée de commander au moins un dispositif médical (DG), avec
- un dispositif de communication (sK) côté serveur pour la communication avec l'appareil médical (DG) ; et
- un module de circuit intégré (cMC) qui est conçu pour mettre en oeuvre un protocole de sécurité pour établir une liaison de communication sécurisée pour la commande de l'appareil médical (DG), et dans lequel le protocole de sécurité échange des messages d'identification pour l'identification univoque de l'appareil médical (DG) et pour l'identification univoque de l'instance de commande respective (SI) sur l'appareil de commande (SG) entre l'appareil médical (DG) et l'appareil de commande (SG),
l'appareil de commande (SG) présentant une **interface utilisateur (GUI)**, pour la saisie de signaux de commande (s) pour la commande de l'appareil médical (DG) et/ou pour la sortie de données d'état de l'appareil médical (DG), **l'interface utilisateur (GUI) étant réalisée sur un appareil séparé en dehors de l'appareil de commande (SG)** , qui est en échange de données bidirectionnel avec l'appareil de commande (SG), l'interface utilisateur (GUI) ne pouvant communiquer avec l'appareil de commande (SG) distant - en particulier par l'intermédiaire de l'appareil médical - que lorsqu'un **capteur de position détecte que l'interface utilisateur (GUI)** se trouve **à proximité spatiale de l'appareil médical** ; et
toutes les unités étant en échange de données via au moins un réseau (NW).

2. Système de commande selon la revendication 1, dans lequel le dispositif de commande (SG) est activé pour exécuter le protocole de sauvegarde en recevant un message d'initialisation (BROADC1) sur le dispositif de commande (SG).

3. Procédé de sécurisation pour l'établissement d'une liaison de communication sécurisée entre au moins un appareil médical (DG), en particulier un appareil de dialyse, et un appareil de commande (SG) distant destiné à être utilisé dans un système de commande selon la revendication 1 ou 2, dans lequel l'appareil de commande (SG) comprend au moins une instance de commande (SI) qui est chargée respectivement de la commande d'au moins un appareil médical (DG), comprenant les étapes de procédé suivantes
- l'exécution (S2) d'un protocole de sécurité sur l'appareil de commande (DG) et l'appareil médical (DG) pour établir une liaison de communication sécurisée pour la commande de l'appareil médical (DG), le protocole de sécurité échangeant des messages d'identification pour l'identification univoque de l'appareil médical (DG) et pour l'identification univoque de l'instance de commande respective (SI) sur l'appareil de commande (SG) avec l'appareil médical (DG).

4. Procédé de sécurisation selon la revendication 3, comprenant l'étape suivante :
- la détection (S1) d'un signal de déclenchement (t) sur le dispositif médical (DG) pour déclencher l'exécution du protocole de sauvegarde.

5. Procédé de sécurisation selon la revendication 3 ou 4, comprenant les étapes suivantes :
- l'envoi (S21) d'un message d'initialisation (BROADC1) contenant une identification de dispositif unique pour le dispositif médical (DG) depuis le dispositif médical (DG) vers le dispositif de commande (SG) ;
- la réception (S22) du message d'initialisation (BROADC1) sur le dispositif de commande (SG), le message d'initialisation (BROADC1) comprenant un code d'équipement et/ou des données de calibrage représentant l'équipement technique du dispositif médical (DG)
- de la part du dispositif de commande (SG) : envoi (S23) d'un premier message d'accusé de réception (ACK1) au dispositif médical (DG) avec une identification de l'instance de commande associée (SI) en réponse au message d'initialisation (BROADC1) ;
- la réception (S24) du premier message d'accusé de réception (ACK1) sur le dispositif médical (DG) ; et
- de la part du dispositif médical (DG) : l'envoi (S25) d'un deuxième message d'accusé de réception (ACK2) au dispositif de commande (SG) en réponse au premier message d'accusé de réception (ACK1) reçu.

6. Procédé de sécurisation selon la revendication 5, dans lequel le dispositif de commande (SG) est configuré après la réception du message d'initialisation (BROADC1) en réponse au message d'initialisation (BROADC1) reçu.

7. Procédé de sécurisation selon la revendication 5 ou 6, dans lequel plusieurs instances de commande (SI) sont formées sur le dispositif de commande (SG) ou plusieurs dispositifs de commande (SG) se trouvent dans un réseau et dans lequel le message d'initialisation (BROADC1) est un message de diffusion à toutes les instances de commande (SI).

8. Procédé de sécurisation selon l'une des revendications 5 à 7, dans lequel le message d'initialisation (BROADC1) comprend un code d'équipement et/ou des données d'étalonnage représentatifs de l'équipement technique du dispositif médical (DG).

9. Procédé de sécurisation selon l'une des revendications 5 à 8, dans lequel, après l'exécution du protocole de sécurité, un résultat de couplage est affiché sur une interface utilisateur de l'appareil de commande (SG) et/ou de l'appareil médical (DG), ou le résultat de couplage est signalé au moyen d'une activation d'un module matériel.

10. Procédé de sécurisation selon l'une quelconque des revendications 5 à 9, dans lequel l'exécution du protocole de sécurité comprend :
- du côté du dispositif de commande (SG) : évaluer si un deuxième message d'accusé de réception (ACK2) a été reçu en réponse à un premier message d'accusé de réception (ACK1) reçu sur le dispositif de commande (SG) dans une période de temps d'attente prédéfinie, et dans la négative : émettre un message d'échec de couplage sur le dispositif de commande (SG) et/ou dans l'affirmative : émettre un message de couplage sur le dispositif de commande (SG).
